# EUROPEAN PATENT SPECIFICATION

(11) **EP 0 596 028 B1**
(45) Date of publication and mention of the grant of the patent: **02.04.1997**
(21) Application number: 92917193.2
(22) Date of filing: 17.07.1992
(51) Int. Cl.: C12Q 1/68

(54) **METHOD FOR DETECTION OF MUTATIONS**
VERFAHREN ZUM NACHWEIS VON MUTATIONEN
PROCEDE DE DETECTION DE MUTATIONS

(30) Priority: 19.07.1991 US 732219
(43) Date of publication of application: 11.05.1994
(73) Proprietor: UPSTATE BIOTECHNOLOGY, INC., Lake Placid, NY 12946 (US)
(72) Inventor: WAGNER, Robert, E., Jr., Vermontville, NY 12989 (US); RADMAN, Miroslav, F-75013 France (FR)
(74) Representative: Harvey, David Gareth
(86) International application number: US9206045
(87) International publication number: WO9302216

(56) References cited:
- EP-A- 0 310 251
- GB-A- 2 179 735
- NUCLEIC ACIDS RESEARCH. vol. 16, no. 16, 1988, ARLINGTON, VIRGINIA US pages 7843 - 7853 J.JIRICNY ET AL. 'Mismatch-containing oligonucleotide duplexes bound by the E. coli mutS-encoded protein' cited in the application
- Genes 3rd ed., Benjamin Lewin, editor; John Wiley & Sons, publishers, 1987, pp. 320 and 574.

## Description

### Background Description

Assays capable of detecting the presence of a particular nucleic acid molecule in a sample are of substantial importance in the prediction and diagnosis of disease, forensic medicine, epidemiology and public health. Such assays can be used, for sample, to detect the presence of a mutant gene in an individual, allowing determination of the probability that the individual will suffer from a genetic disease. The ability to detect a mutation has taken on increasing importance in early detection of cancer or discovery of susceptibility to cancer with the discovery that discrete mutations in cellular oncogenes can result in activation of that oncogene leading to the transformation of that cell into a cancer cell (Nishimura, S. et al., Biochem. J. 243:313-327 (1987); Bos, J.L., Cancer Res. 49:4682-4689 (1989)).

Nucleic acid detection assays can be based on any of a number of characteristics of a nucleic acid molecule, such as its size, sequence, susceptibility to digestion by restriction endonucleases, etc. The sensitivity of such assays may be increased by altering the manner in which detection is reported or signaled to the observer. Thus, for example, assay sensitivity can be increased through the use of detectably labeled reagents. A wide variety of such labels have been used for this purpose, such as enzyme labels (Kourilsky et al., U.S. Patent 4,581,333), radioisotopic labels (Falkow et al., U.S. Patent 4,358,535; Berninger, U.S. Patent 4,446,236), fluorescent labels (Albarella et al., EP 144914), chemical labels (Sheldon III et al., U.S. Patent 4,582,789; Albarella et al., U.S. Patent 4,563,417), modified bases (Miyoshi et al., EP 119448), and the like.

Several nucleic acid detection systems have been designed to detect specific mutations, and involve a combination of probes and additional binding partners which act as reporters. Paau et al., U.S. Patent 4,556,643 discloses a polynucleotide probe having a target-specific sequence and a protein-binding sequence. In most cases, such a protein-binding sequence is an extra segment of DNA ligated to the target-specific segment. The binding proteins used can be part of a protein-marker complex, such as a protein-enzyme complex (col. 7, lines 7-10). Disclosed binding protein were DNA-modifying enzymes, polymerases, lac repressor, or antigenic DNA sequences (col. 7, lines 10-29). A typical example of a probe taught by this reference (Example I, col. 12 and 13) is a cDNA molecule (capable of hybridizing to the target DNA sequence) to which has been ligated T7 promoter (the protein binding sequence). After hybridization of this probe, an E. coli RNA polymerase is added as the binding protein and binds to the T7 promoter. The presence of the bound RNA polymerase is detected by a rabbit antiserum specific for the polymerase followed by peroxidase-conjugated goat-anti rabbit second antibody, and a peroxidase-anti-peroxidase detection system.

Mundy, U.S. Patent 4,656,127, discloses methods for detecting a mutation of a specific nucleotide base which require knowledge of the sequence in the region of interest (col. 1, lines 52-43), and have the advantage of not requiring that the mutation be at a restriction site. A linear probe complementary to part of the nucleic acid chain is provided which extends in one direction from the mutational site. Following hybridization, a nucleotide derivative is added which joins the end of the probe depending on whether a particular base sequence is present or absent. The single stranded portion is digested. The presence or absence of the probe is then detected (col. 2, lines 3-22). The reference envisions a thionucleotide as the nucleotide derivative (col. 4, lines 46-51), which is detected by virtue of being detectably labeled.

Kato et al., European Patent Publication 407,789, discloses methods for detecting a nucleic acid based on hybridization with a labeled probe. Among the nonradioactive labels mentioned are enzymes, and fluorescence, utilizing a biotin-avidin system (p. 2, lines 36-39). The exemplified label is biotin-labeled dUTP which is synthetically incorporated into the probe.

A number of methods designed to detect base-pairing mismatches in nucleic acid hybrids is known in the art. Earlier methods were based on enzymatic cleavage of mismatches (Gibbs, R. et al., Science 236:303-305 (1987)). These methods involve a step of enzymatically cleaving the mismatched DNA hybrid. One drawback of these methods is that they do not reliably detect all mismatches. A recently described chemical method for cleavage of mismatched DNA (Cotton, R.G. et al., Proc. Natl. Sci. USA 85:4397-4401 (1988); Cotton, R.G., Nuc. Acids Res. 17:4223-4233 (1989)) is based on chemical cleavage at a mismatch site in DNA-DNA heteroduplex, using a number of agents, in particular osmium tetroxide and hydroxylamine. In this method DNA probes are prepared by restriction enzyme cleavage of DNA of interest. Plasmid DNA containing the sequence of interest is hybridized to labeled probe DNA (either end-labeled or internally labeled with ³²P). Hydroxylamine chemically modifies mismatched cytosines; osmium tetroxide modifies mismatched thymines. Piperidine is then used to cleave the DNA at the modified sites, followed by polyacrylamide gel electrophoresis (PAGE) and autoradiography to identify the cleavage products. This method is said to have the advantage of detecting all possible single base pair mismatches because, the method also results in cleavage at a matched base pair in the vicinity of a mismatch.

Caskey and coworkers disclose a method for localizing a mutation which utilizes PCR-amplified cDNA as a source of template for the mismatch cleavage reaction. This technique was applied in studying ornithine transcarbamoylase (OTCase) deficiency patients to map mutations.

Kung et al., U.S. Patent 4,963,658, discloses detecting single stranded DNA (ssDNA) by binding with a high-affinity ssDNA-binding protein, such as a topoisomerase or a DNA unwinding protein (col. 2, lines 44-47) which itself can be bound to a label, such as β-D-galactosidase (col. 7, line 1).

The desire to increase the utility and applicability of presently available assays is often frustrated by assay sensitivity as well as complexity and cost. Hence, it would be highly desirable to develop more sensitive, as well as simple and relatively inexpensive, assays for detection of alterations in DNA.

### Summary of the Invention

The present invention relates to a method of detecting mutations in a nucleic acid sequence which involve as little as one base change in the sequence. The extent to which a target polynucleotide occurs in a sample can also be determined. The present method is based on the use of a mismatch binding protein, as exemplified by the MutS protein of E. coli and Salmonella. As used herein, the term target polynucleotide is a nucleic acid sequence which is to be detected. The target polynucleotide sequence which does not contain the mutation (the unaltered form) is referred to as the non-mutated target polynucleotide or the non-mutated target sequence. The target polynucleotide sequence containing a mutation or alteration is referred to as mutated target polynucleotide. The present method is useful for diagnosing a variety of important disease states or susceptibilities, such as, by detecting the presence of a mutated oncogene. The method shares the efficiency advantages of PCR-based methods, but is simpler and cheaper to perform. The inventors have also devised a kit for carrying out the method of this invention.

The DNA being analyzed can be of any type such as DNA obtained from blood cells, sperm or tissue of an organism, preferably a human. The method of this invention does not require elaborate or expensive apparatus, as is used in PCR, and can be carried out by a technician without sophisticated training. The method can be internally controlled, such that the test sample and control DNA are subject to the same treatments. Finally the method can be designed to yield simple, easily interpretable results, such as a colored "plus" or "minus" sign on a membrane filter.

This invention is based on two key elements. The first is the existence of proteins which bind to a double stranded nucleic acid heteroduplex containing a mispaired or unpaired base. Well-known examples of such mismatch-binding proteins are bacterial proteins, such as the MutS protein in E. coli, which function in DNA repair. The second element is the relative ease of isolating and sequencing specific genes and their mutant alleles.

Thus, the present invention is directed to a method for determining whether a mutation is present in the sequence of a single stranded polynucleotide (or target polynucleotide) in a sample. That is, it is a method of detecting a mutated target polynucleotide or, alternatively, of determining whether a mutation is present (or absent) in a polynucleotide (DNA or RNA).

In the method, a sample to be analyzed for the mutated target polynucleotide is incubated with a single-stranded polynucleotide hybridization partner which is at least one single stranded base sequence complementary to the sequence of the non-mutated target polynucleotide. The hybridization partner and polynucleotides to be assessed for the mutated target polynucleotide (e.g., a DNA or RNA sample) are incubated under conditions suitable to cause hybridization of the hybridization partner to any mutated or non-mutated target polynucleotide which may be present in the sample. Hybridization of complementary sequences results in formation of a hybrid of the hybridization partner and the target polynucleotide. The hybrid formed is combined or contacted with a mismatch-binding protein under conditions suitable to cause binding of the mismatch-binding protein to mutated target polynucleotide. This results in binding of mismatched binding proteins to hybrids in which the target polynucleotide is mutated target polynucleotide. The detection of the presence of the mismatch-binding protein bound to the hybrid is indicative of the presence of a mutation in the sequence of the polynucleotide of the sample (i.e., is indicative of the presence of the mutated target polynucleotide).

Preferably, in the above mentioned, the hybridization partner is DNA, for example, cDNA or a synthetic oligonucleotide. The target polynucleotide may be DNA or RNA. A preferred RNA is mRNA.

In the above method a preferred mismatch-binding protein is the MutS protein or a functional derivative thereof.

In one embodiment of the method, the sample is a biological fluid which has been subjected to conditions sufficient to release and denature (render single stranded) any nucleic acid present therein. Optionally, the released nucleic acid is subjected to a step of restriction endonuclease cleavage prior to the denaturation.

In one embodiment, the polynucleotide hybridization partner is immobilized on a solid support and the resulting solid support-bound hybridization partner is incubated with a sample of polynucleotide to be assessed for the presence of mutated target polynucleotide. A preferred solid support is a nitrocellulose membrane.

The detection step preferably comprises adding a detectably labeled first binding partner which binds to the mismatch-binding protein. The first binding partner may be, for example, an antibody specific for the mismatch-binding protein or an enzyme which catalyzes generation of a colored reaction product, which is detected.

In another embodiment, the detection step comprises adding a first binding partner which binds to the mismatch-binding protein and is not detectably labeled, and a second binding partner which binds to the first binding partner and detects the presence of the first binding partner. The second binding partner may either itself be detectably labeled or a detectably labeled third binding partner which binds to the second binding partner may be used. A preferred second binding partner is an antibody for the first binding partner. In another embodiment, the first binding partner is a MutL protein or a functional derivative thereof, preferably a fusion protein between MutL and a second protein or peptide capable of being detected. A preferred second protein in the fusion protein is beta-galactosidase.

In another embodiment, the mismatch-binding protein is one component of a fusion protein, in which the second component is a second protein or peptide capable of being detected, either directly (i.e, it is itself detectable) or indirectly (i.e., through the use of a reagent which is detectable). In this embodiment, the detection step preferably comprises adding a detectably labeled binding partner which binds to the second protein or peptide present in the fusion protein. The second protein may be an enzyme capable of catalyzing the generation of a colored reaction product from a chromogenic enzyme substrate. The detection step comprises providing the chromogenic substrate and visualizing the color reaction product. A preferred second protein is beta-galactosidase.

The present invention is further directed to a method for detecting (distinguishing) a mutated from a non-mutated sequence of a single stranded target mammalian polynucleotide in a sample. In this method, a sample of mammalian polynucleotide (DNA or RNA) is obtained, using known methods. For example, DNA or RNA can be obtained from a cell, treated to render it single stranded and incubated with a single-stranded polynucleotide hybridization partner which is at least one single stranded base sequence which is complementary to the sequence of the non-mutated target mammalian polynucleotide. The DNA or RNA and the hybridization partner are incubated under conditions suitable to cause hybridization of the hybridization partner to any mutated or non-mutated target polynucleotide which may be present in the sample. Hybridization of complementary sequences results in formation of a hybrid of the hybridization partner and the target polynucleotide. The hybrid formed is combined or contacted with a mismatch-binding protein under conditions suitable to cause binding of the mismatch-binding protein and hybrids, in which target polynucleotide is mutated target polynucleotide. The detection of the mismatch-binding protein bound to the hybrid is indicative of the presence of a mutation in the sequence of the mammalian polynucleotide of the sample (i.e., is indicative of the presence of the mutated target polynucleotides).

The present invention includes a kit useful for detecting a mutated target polynucleotide (distinguishing mutated target polynucleotide from non-mutated target polynucleotide sequence) in a sample. The kit includes the following:
(a) a first container containing a single-stranded polynucleotide hybridization partner comprising at least one single stranded base sequence which is complementary to the non-mutated sequence of the target polynucleotide (i.e., is complementary to the non-mutated target polynucleotide);
(b) a second container containing a mismatch-binding protein; and
(c) a third container or a plurality of containers containing a reagent or reagents capable of detecting the binding of the mismatch-binding protein to the hybrid.

In the above kit, the mismatch-binding protein is preferably MutS or a functional derivative thereof. The reagent or reagents preferably comprise at least a detectably labeled first binding partner capable of binding to the mismatch-binding protein. A preferred first binding partner is an antibody specific for the mismatch-binding protein.

In an alternative kit embodiment, the reagent or reagents comprise a first binding partner which is not detectably labeled, the first binding partner being capable of binding to the mismatch-binding protein, and a second binding partner, such as an antibody, capable of binding to the first binding partner. The second binding partner may be detectably labeled. Also intended is a kit wherein the second binding partner is not labeled and a detectably labelled third binding partner capable of binding to the second binding partner is included.

In a preferred kit embodiment, the first binding partner is a MutL protein or a functional derivative thereof, such as a fusion protein between MutL and a second protein or peptide capable of being detected, preferably β-galactosidase. A second binding partner is capable of binding to the MutL protein or its fusion partner protein, for example an antibody specific for *β*-galactosidase.

In the above kit the hybridization partner is preferably immobilized on a solid support. A preferred solid support is a nitrocellulose membrane.

In another kit embodiment, the mismatch-binding protein is a fusion protein fused with an enzyme and the reagent comprises a chromogenic substrate for the enzyme.

### Brief Description of the Drawings

Figure 1 is a schematic diagram showing the hybridization of hybridization partners in the form of oligonucleotide or cDNA hybridization partners with the test or "target" DNA. The lower portion of the figure shows the configuration of mismatch containing hybrids. Where a cDNA has hybridized to a genomic DNA fragment, non-hybridizing portions of the genomic DNA (introns) are shown to loop out.

Figure 2 is a schematic representation of the mutation detection assay of the present invention. In this particular embodiment, the presence of the mismatch-binding protein is detected through the use of a first binding partner, an antibody specific for the mismatch-binding protein. This antibody may be detectably labeled or may be detected by a detectably labeled reagent (lower right). Alternatively, the detection step can be amplified using a second binding partner, here an antibody specific for the first antibody ("Anti-Ab") (lower left).

Figure 3 is a schematic diagram of a particular assay format, as described in the Examples, wherein positive and negative controls are deposited on a nitrocellulose filter such that a visible "minus" sign is obtained if no mismatch is present and a visible "plus" sign is obtained if the target DNA contains a mutation (mismatch).

Figure 4 shows results of mismatch detection in heteroduplexes annealed in solution.

Figure 5 shows results of mismatch detection in heteroduplexes annealed to immobilized oligonucleotides.

Figure 6 shows results of mismatch detection in heteroduplexes annealed in the presence of competing oligonucleotides.

### Description of the Preferred Embodiments

The present inventors conceived of a new, broadly applicable and relatively simple method for detecting alterations, even as a small as a single base change, in a DNA sequence. Bacterial DNA mismatch repair mechanisms employ a family of proteins, including a protein, which recognizes and binds to double stranded DNA containing a base pair mismatch (for reviews, see Radman, M. et al., Annu. Rev. Genet. 20:523-538 (1986); Radman, M. et al., Sci. Amer., August 1988, pp. 40-46; Modrich, P., J. Biol. Chem. 264:6597-6600 (1989)). The MutS protein was identified as such a component of the E. coli mismatch repair system (Lahue, R.S. et al., Science 245:160-164 (1989); Jiricny, J. et al., Nucl. Acids Res. 16:7843-7853 (1988); Su, S.S. et al., J. Biol. Chem. 263:6829-6835 91988); Lahue, R.S. et al., Mutat. Res. 198:37-43 (1988); Lahue, R.S. et al., Proc. Natl. Acad. Sci. USA 84:1482-1486 (1987); Dohet, C. et al., Mol. Gen. Genet. 206:181-184 (1987); Jones, M. et al., Genetics 115:605-610 (1987); Su, S.S. et al., Proc. Natl. Acad. Sci. USA 83:5057-5061 (1986); Dohet, C. et al., Proc. Natl. Acad. Sci. USA 82:503-505 (1985); Choy, H.E. et al., Mutat. Res. 142:93-97 (1985); Jones, M. et al., Mol. Gen. Genet. 184:562-563 (1981)). Analogous proteins are known in other bacterial species including MutS in Salmonella typhimurium (Lu, A.L. et al., Genetics 118:593-600 (1988); Haber, L.T. et al., J. Bacteriol. 170:197-202 (1988); Pang, P.P. et al., J. Bacteriol. 163:1007-1015 (1985)) and the hexA protein of Streptococcus pneumoniae (Priebe, S.D. et al., J. Bacteriol. 170:190-196 (1988); Haber et al., supra).

Purified MutS protein binds DNA containing mispaired bases, but does not bind DNA without mismatches or single-stranded DNA. The MutS-DNA interaction does not result in any degradation or modification of the DNA.

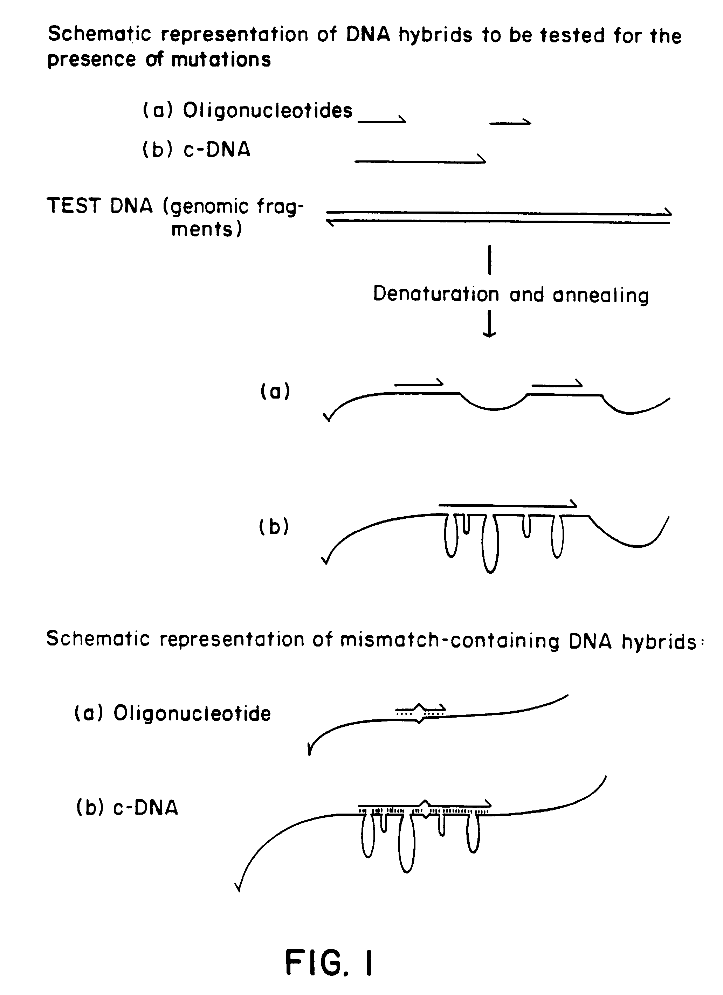

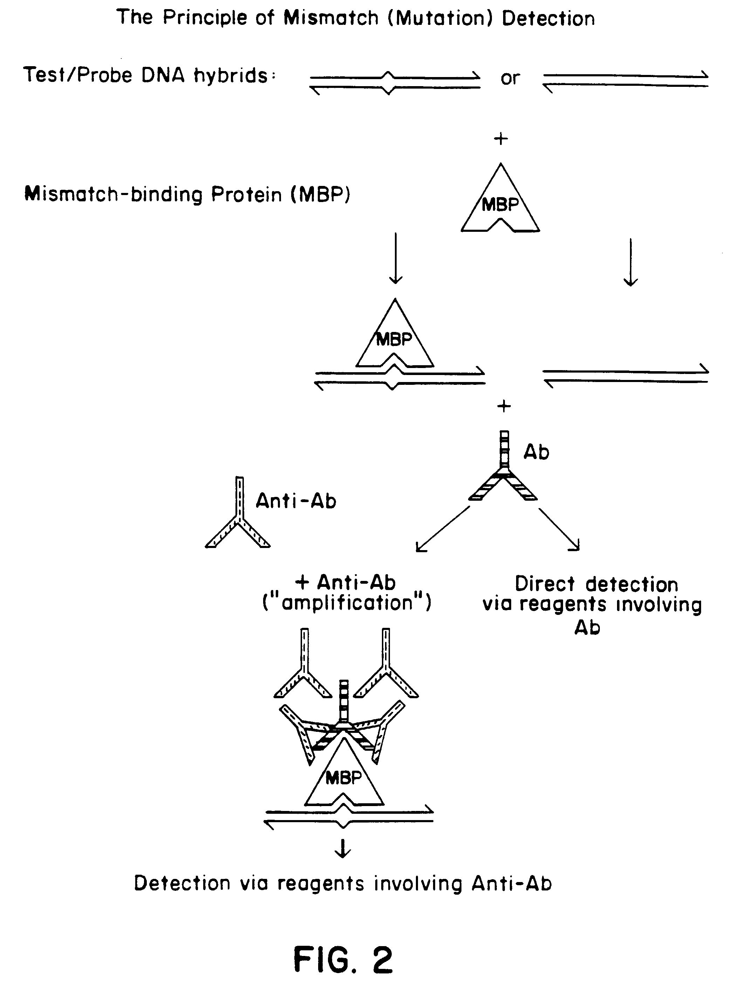

This method is based upon detection of a mismatched DNA heteroduplex formed when a strand of mutant DNA and a "complementary" strand of wild-type DNA hybridize (see Figure 1). The presence of the mismatch is detected in a highly specific manner by first allowing a mismatch-binding protein, such as the MutS protein E. coli, to bind to the DNA duplex. The presence of the bound mismatch-binding protein is then detected in any of a number of ways, for example, by using a detectably labeled antibody specific for the mismatch-binding protein, e.g., an anti-MutS antibody. This method stands in stark contrast to methods of the prior art which employ mismatch cutting nuclease enzymes capable of breaking DNA at or near a mispaired base pair. For a schematic illustration of this approach, see Figure 2.

The method of the present invention permits the detection of the presence of a particular "target" nucleic acid molecule or nucleic acid molecule or polynucleotide in a sample. Such samples are typically biological samples, such as, for example, blood, sperm, stool, sera, urine, saliva, culture medium, etc., as well as non-biological samples, such as, for example, waste or drinking water, milk or other foods, air, etc.

The target polynucleotide or nucleic acid molecule detected by the method of the present invention may be either DNA or RNA, but is preferably DNA. When the hybridization partner is cDNA, the target nucleic acid may be either DNA or mRNA, the mRNA being present in higher copy number. As used herein, the term "target nucleic acid", "target polynucleotide" or "target sequence" refers to a nucleic acid which contains a sequence of interest to be detected an/or quantified. It may include the wild type sequence, in which case it is referred to as the wild type or non-mutated target polynucleotide or non-mutated target sequence. Alternatively, it may contain an alteration or mutation (mutated from wild type), in which case it is referred to as mutated target polynucleotide. A target sequence in the target nucleic acid must be sufficiently long to hybridize with a probe DNA (hybridization partner) even if there is an alteration or mutation within the polynucleotide. A target sequence will be preferably greater than 30 bases to be detected by the present method. In one embodiment, the target nucleotide sequence must be known, in order to permit construction of a "hybridization partner" polynucleotide or oligonucleotide having a sequence with the requisite mismatch relative to the target sequence to permit the binding of the mismatch-binding protein and its subsequent detection.

In another embodiment, a cDNA molecule is used as the hybridization partner. In this case, it is not necessary to know in advance the target nucleotide sequence. Upon hybridization of cDNA with a genomic DNA fragment, introns not represented in the cDNA form unpaired loops (see Figure 1), which are neither detected by the mismatch-binding protein nor interfere in its binding to single base mismatches or to mismatches caused by addition or deletion of 1-4 base pairs.

The method of the present invention has the advantage of permitting the detection of a mutant target nucleic acid molecule (either DNA or RNA) without requiring the amplification of such a molecule, as is common in a number of more recently devised mutation detection assays. The present invention accomplishes this goal through the construction and use of one or more hybridization partner oligonucleotide or polynucleotide molecules, including cDNA molecules, having sequences complementary to one or more wild-type target nucleotide sequences. The hybridization partner is capable of hybridizing to a target nucleic acid molecule, such that, when a base pair mismatch or unpaired base occurs, this mismatch or unpaired base is recognized by the mismatch-binding protein.

In a preferred embodiment, the hybridization partner is a single-stranded nucleic acid molecule, most preferably, single-stranded DNA.

The target nucleic acid molecule may, for example, be DNA or RNA from eukaryotic cells, prokaryotic cells, viruses, viroids, or the like. Preferably, the target nucleic acid molecule is of mammalian origin, most preferably, of human origin. Indeed, there are no constraints to the nucleotide sequence, or origin, of the "target" nucleic acid molecule.

Small frameshift mutations, either additions or deletions, also result in a sequence which will pair with the wild-type sequence in a manner such that the mismatch-binding protein will bind. For example, it is known that both the E. coli MutS-based repair system (Radman, M. et al., Annu. Rev. Genet. 20:523-538 (1986)) and the Streptococcus pneumoniae Hex mismatch repair system act on mismatches resulting from the addition or deletion of a single base. Additions or deletions of two or three base pairs, and to a lesser extent of four base pairs are also repaired, whereas an addition or deletion of five base pairs is not. Thus, the methods of the present invention are useful for detecting small addition or deletion mutations of between one of four bases.

In an assay to detect a particular single base mutation in double stranded (ds) DNA, it is preferable to use two wild-type hybridization partner sequences which, but for a single base, are complementary to each of the two mutant DNA strands, since the sensitivity of the assay will be increased by two-fold compared to the use of only one hybridization partner. However, it is also possible to use a single hybridization partner sequence complementary to only one of the two DNA strands because the assay is sufficiently sensitive such that the two-fold decrease in sensitivity (compared to using two hybridization partners) does not detract from the utility of the method.

Because a mismatch-binding protein, for example MutS, does not bind all possible mismatches with equal efficiency (Dohet, C. et al., 1985, supra; Jones, M. et al., 1987, supra), it may be necessary for certain target sequences to prepare hybridization partners complementary to both strands in the region of interest in order to create both of the two possible mismatches from a given dsDNA wild-type sequence in order to detect at least one of the two mutant sequences.

The hybridization partner can be a single continuous polynucleotide segment, complementary to a larger target sequence wherein more than a single base pair is mismatched. Alternatively, two mutations on a single fragment of target DNA that are separated by about 30 or more bases can be detected by using two or more individual hybridization partners, each of which hybridizes with a separate sequence on the single target fragment.

In one embodiment, the region of the hybridization partner complementary to the target sequence can be flanked at the 3' or 5' termini by non-hybridizable sequences, provided that the hybridizable portion forms a stable heteroduplex of at least about 30 nucleotides wherein the mismatch-binding protein can recognize a mismatch and bind.

The RNA or DNA hybridization partner can be prepared by a variety of well-known means. Most preferably, an oligonucleotide hybridization partner is prepared by chemical oligonucleotide synthesis using conventional methods. Techniques for synthesizing oligonucleotides are disclosed, for example, by Wu, R., et al., Prog. Nucl. Acid. Res. Molec. Biol. 21:101-141 (1978)). Alternatively, an RNA hybridization partner can be isolated as a natural product of cells, for example, as an mRNA molecule. RNA or cDNA hybridization partners can also be prepared by recombinant DNA methods. (See for example, Green et al., Cell 32:681 (1983)). DNA hybridization partners can be prepared from any of a variety of sources readily apparent to one of skill in the art, for example by restriction endonuclease cleavage or chemical cleavage of naturally occurring DNA.

Given the fact the E. coli MutS binds to heteroduplexes of 16 bases (Jiricny et al., supra), and that the size of the heteroduplex protected by MutS in a footprinting assay is as little as 8-12 bases (Su, S-S. et al., Proc. Natl. Acad. Sci. USA 83:5057-5061 (1986)), the lower limit for the size of the hybridization partner of the present invention is about 10 nucleotides. To increase fidelity of hybridization while trying to keep synthetic costs down, a larger hybridization partner is preferred, preferably from about 20 to about 100 nucleotides. Given the economics of oligonucleotide synthesis, a more preferred oligonucleotide hybridization partner will have from about 20 to about 40 nucleotides. There is no upper limit on the size of the hybridization partner, since recombinant methods can be used for its production, as described herein. Thus, in another embodiment, an intact cDNA molecule is the hybridization partner.

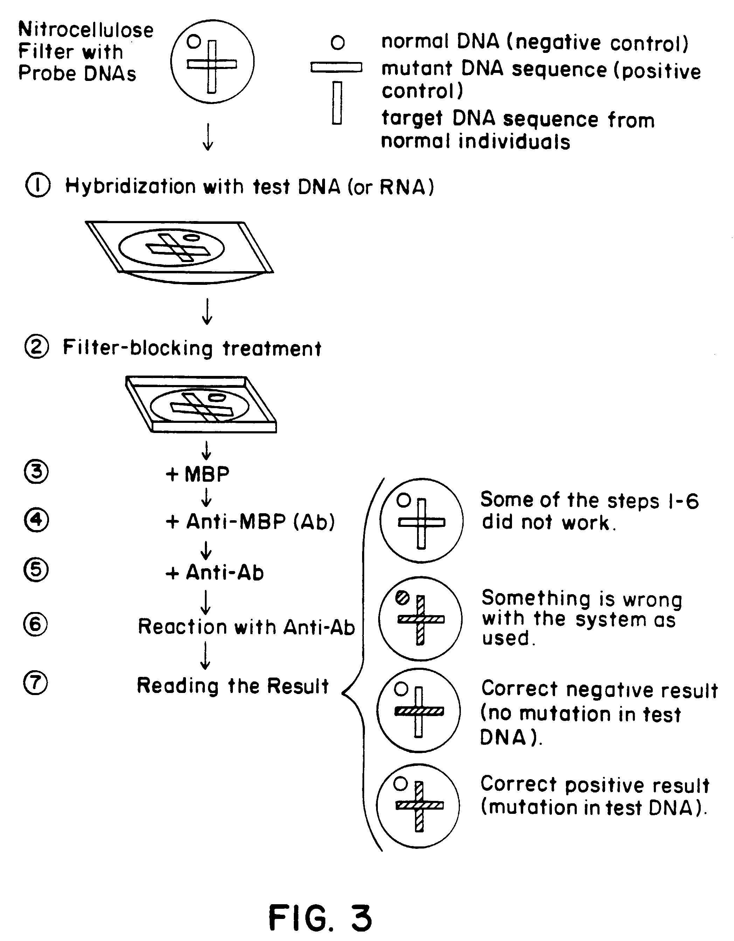

For use in the methods of the present invention, the hybridization partner sequence is in immobilizable form or, more preferably, is immobilized on a solid phase support or carrier (see Figure 3). An immobilizable form of the hybridization partner will be one in which can be conveniently rendered immobilized, generally subsequent to the hybridization reaction. The means by which the hybridization partner is ultimately immobilized is not critical to the present invention, and any available approach can be taken as long as the hybrids formed between the hybridization partner and the target nucleic acid sequence are rendered immobilized through a property of the hybridization partner.

When introduction into the hybridization reaction in an immobilized form, the hybridization partner can be in any appropriate form that enables it, and any components of the reaction mixture that have become bound to it, to be subsequently isolated or separated from the remaining mixture. Separation may be accomplished by centrifugation, filtration, chromatography, decanting or the like.

One of ordinary skill in the art will appreciate the variety of compositions and configurations of an immobilized hybridization partner useful according to the present invention. For example, the hybridization partner can be aggregated or otherwise precipitated, attached to an insoluble material, polymer or support, or entrapped in a gel such as agarose or polyacrylamide. (Meth. Enzymol., 12B:635 (1968); Proc. Natl. Acad. Sci. USA 67:807 (1970)). Most preferred is a solid support to which the hybridization partner is attached or fixed by covalent or noncovalent bonds. Preferably, noncovalent attachment is by adsorption using methods that provide for a suitably stable and strong attachment. This can also be accomplished by means of an amino modifier on the hybridization partner; the amino modifier is used to "hook" or attach the hybridization partner to a solid support (e.g., a membrane or filter). The means for attaching a desired oligonucleotide or polynucleotide to a selected solid support are well known to those of ordinary skill in the art.

By "solid phase support" is intended any support capable of binding an oligo- or polynucleotide. Well-known supports or carriers include natural and modified celluloses, such as nitrocellulose, polystyrene, polypropylene, polyethylene, dextran, nylon, polyacrylamides, and agaroses. The support material may be virtually any possible structural configuration, as long as the immobilized hybridization partner can hybridize to the target nucleic acid molecule and the mismatch-binding protein can subsequently bind to the hybridization partner-target polynucleotide hybrid. Thus, the support configuration can include microparticles, beads, porous and impermeable strips and membranes, the interior surface of a reaction vessel such as test tubes and microtiter plates, and the like. Preferred supports include nitrocellulose disks or strips. Those skilled in the art will know many other suitable carriers for binding the oligo- or polynucleotide hybridization partners, or will be able to ascertain these by routine experimentation.

A preferred method for adsorbing the hybridization partner onto nitrocellulose membranes involves saturating a solution of the hybridization partner with sodium iodide and spotting or filtering aliquots onto the membrane (Bresser et al., DNA 2:243 (1983)). Alternatively, the hybridization partner can be treated with glyoxal (less than 1 M), and then adsorbed to the membrane. The hybridization partner may be fixed by baking at around 80°C under vacuum for about 2-3 hours (Thomas, P.S., Meth. Enzymol. 100:255).

Covalent immobilization of the oligonucleotide hybridization partners can also be accomplished. Numerous support materials and coupling methods useful in covalent immobilization are known. Examples include coupling to phosphocellulose through phosphate groups activated by carbodiimide or carbonyldiimidazole (Bautz, E.K.F. et al., Proc. Natl. Acad. Sci. USA 48:400-408 (1963); Shih, T.Y. et al., Biochemistry 113:3411-3418 (1974)), or reactions of diazo groups on m-diazobenzoyloxymethyl cellulose with G and T residues of the hybridization partner (Noyes, B.E. et al., Cell 5:301-310 (1975); Reiser, J. et al., Biochem. Biophys. Res. Comm. 85:1104-1112 (1978)). Polysaccharide supports can be coupled through phosphodiester links formed between terminal phosphate of the oligonucleotide and hydroxyl moieties of the support by water soluble carbodiimide activation (Richwood, D., Biochim. Biophys. Acta 269:47-50 (1972)), or by coupling nucleophilic sites on the oligonucleotide with a cyanogen bromide-activated support (Arndt-Jovin, D.J. et al., Eur. J. Biochem. 54:411-418 (1975)). Further, 3' hydroxyl terminus of the hybridization partner can be oxidized by periodate and coupled by Schiff base formation with supports bearing amine or hydrazide groups (Gilham, P.T., Method. Enzymol. 21:191-197 (1971); Hansske, H.D., et al., Method. Enzymol. 59:172-181 (1979)). Supports having nucleophilic sites can be reacted with cyanuric chloride and then with the polynucleotide (Hunger, H.D. et al., Biochim. Biophys. Acta 653:344-349 (1981)).

In general, any method can be employed for immobilizing the hybridization partner, provided that a sequence complementary to the target sequence is available for hybridization to a sample nucleic acid. Particular methods or materials are not critical to the present invention.

An alternative to employing directly immobilized hybridization partner is to use an immobilizable form, allowing performance of the hybridization step in solution where the kinetics are more rapid. Typically, in such an embodiment, one would use a hybridization partner comprising a reactive site capable of forming a stable covalent or noncovalent bond with a reaction partner and obtain immobilization by exposure to an immobilized form of the reaction partner. Preferably, such reactive site in the hybridization partner is a binding site such as a biotin or hapten moiety which is capable of specific noncovalent binding with a binding substance such as avidin or an antibody which serves as the immobilized reaction partner.

Essentially any pair of substances can comprise the reactive site/reactive partner pair which exhibit an appropriate affinity for interacting to form a stable bond, that is a linking or coupling between the two which remains substantially intact during the subsequent assay steps, principally the separation and detection steps. The reactive site on the hybridization partner is referred to as a "binding site" and the reaction partner is referred to as a "binding substance" with which it forms a covalent or noncovalent bond or linkage.

In one embodiment, the binding site is preferably present in a nonhybridizable portion of the hybridization partner and is preferably a result of a chemical modification of the hybridization partner. Examples of binding sites existing within the nucleotide sequence are a promoter sequence which can be bound by a promoter protein, an operator sequence which can be bound by a repressor protein, or a modified nucleotide which can be bound by a specific antibody, for example, 5-bromodeoxyuridine or 5-iododeoxyuridine (British Pat. Spec. 2,125,964), thymine glycol (Rajagopalan et al., Radiat. Res. 97:499-510 (1984); Hubbard, K. et al., Radiat. Res. 118:257-268 (1989)).

Binding sites introduced by chemical modification of the oligonucleotide hybridization partner are particularly useful and normally involve linking one member of a specific binding pair to the hybridization partner. Useful binding pairs from which to choose include biotin/avidin (including egg white avidin and streptavidin), haptens (or antigens)/antibodies, carbohydrates/lectins, enzymes/inhibitors, and the like. Where the binding pair consists of a protein member and a nonprotein member, it will normally be preferred to link the nonprotein member to the hybridization partner since the protein member may be unstable under the denaturing conditions of hybridization. Preferable systems involve linking the hybridization partner to biotin or a hapten and employing immobilized avidin or an anti-hapten antibody reagent, respectively.

When the hybridization partner is introduced into the hybridization reaction in an immobilizable form, the subsequent steps of immobilization of the formed duplexes and addition of the mismatch-binding protein and the reagents for detection can proceed in any desired order. Immobilization and addition of the mismatch-binding protein and other reagents can be accomplished by simultaneous addition of the necessary reagents and materials, by sequential addition, with or without intervening washing or separation steps, in either order. Where ordered additions are followed, of course one will take into account the concentrations of the added reagents so as not to oversaturate the formed hybrids and inhibit interaction with the mismatch-binding protein and the detection reagents.

A preferred mismatch-binding protein is characterized by its ability to bind the DNA-DNA (or DNA-RNA or RNA-RNA) base pair mismatched hybrids formed between the hybridization partner and target complementary sample nucleic acids, to the significant exclusion of the single stranded polynucleotides or perfectly matched hybrids. In a preferred embodiment, the intact native MutS protein from E. coli is used. However, as used herein, the term "mismatch binding protein" is intended to encompass a functional derivative of the intact, native protein. By "functional derivative" is meant a "fragment", "variant", "analog", or "chemical derivative" of the protein which retains the ability to bind to a mismatch nucleic acid heteroduplex, in accordance with the present invention.

A "fragment" of a mismatch-binding protein refers to any subset of the molecule, that is, a shorter peptide. A "variant" of the protein refers to a molecule substantially similar to either the entire protein or a DNA-hybrid-binding fragment thereof. A variant of mismatch-binding protein, for example, of MutS, may be prepared by recombinant DNA methods well-known in the art.

A preferred functional derivative of MutS is a homologue of E. coli MutS in another bacterial species, such as the MutS protein of Salmonella typhimurium (Lu, A.L. et al., supra; Habet L.T. et al., supra; Pang, P.P. et al., supra) or the hexA protein of Streptococcus pneumoniae (Priebe S.D. et al., supra; Haber et al., supra). In addition, possible eukaryotic homologues of MutS or HexA can also be used, such as those encoded by the homologous sequences identified in human, mouse or hamster DNA (Shimada, T. et al., J. Biol. Chem. 264:20171 (1989); Linton, J. et al., Molec. Cell. Biol. 7:3058-3072 (1989); Fuji, H. et al., J. Biol. Chem. 264:10057 (1989)).

A "chemical derivative" of the mismatch binding protein contains additional chemical moieties not normally a part of the protein, including additional stretches of amino acids as in a fusion protein. Covalent modifications of the peptide are included within the scope of this invention. Such modifications may be introduced into the molecule by reacting targeted amino acid residues of the protein with an organic derivatizing agent that is capable of reacting with selected side chains or terminal residues. Examples of such derivatization is haptenmodification, wherein a haptenic group such as trinitrophenyl and the like is conjugated to the protein and serves as the target of a detectably labeled binding partner for detection purposes. Alternatively, the protein can be modified by conjugation of biotin groups, and detectably labeled avidin or streptavidin can be used as the binding partner for detection purposes.

A preferred chemical derivative of the mismatch-binding protein is a fusion protein between the mismatch-binding protein and another protein (the "fusion protein partner"), wherein the other protein provides a structural or functional feature which makes it useful for detection of binding. For example, the fusion protein partner may contribute antigenic sites to which an antibody can bind for detection purposes. A fusion protein between the mismatch-binding protein or functional derivative and an enzyme can provide a more direct means of detection, for example, by virtue of the ability of the enzyme portion of the fusion protein to catalyze the formation of a colored reaction product from a chromogenic substrate, as is well-known in the art.

A preferred fusion protein is one between MutS or a functional derivative and the enzyme β-galactosidase. In this embodiment, the presence of the β bound to the solid support can be detected by using a chromogenic substrate of this enzyme. An example of such a substrate is NABG (Naphthol AS-B1-β-d-galactopyranoside).

A fusion protein derivative of the mismatch-binding protein can be prepared using methods well-known in the art (see, for example, Sambrook, J. et al., Molecular Cloning: A Laboratory Manual, 2nd Edition, Cold Spring Harbor Press, Cold Spring Harbor, NY, 1989).

Selection of a protein as being useful for the methods of the present assay can be performed by one of ordinary skill in the art using conventional methods. Thus, for example, in evaluating a source for the presence of a mismatch-binding protein useful in the present invention, one can perform a mismatch-binding assay, such as that described for MutS by Jiricny et al. Preferably, a filter binding assay is used. To prepare the oligonucleotide heteroduplex, an oligonucleotide, preferably of about 16 bases is labeled with ³²P using a kinase reaction and gamma-³²P-ATP using a kinase such as T4-polynucleotide kinase. The 5'-labeled oligonucleotide (which can be stored at -20°C) is then annealed with a complementary oligonucleotide having a single base pair mismatch under standard conditions. The annealed 16 base pair heteroduplex is mixed with an excess of the protein being tested and kept on ice for 30 minutes. The mixture is then applied to a nitrocellulose filter which has been prewetted in the assay buffer. Gentle suction is applied for several seconds, and the filter is washed extensively with ice-cold assay buffer. The filter is then dried in the air, suspended in scintillation fluid and counted. By virtue of the protein sticking to the filter, any counts on the filter can be attributed to binding to the putative mismatch-binding protein. In the absence of such a protein, the labeled oligonucleotide heteroduplex will pass through the filter. Thus, by using such a simple assay, one can easily detect and select a mismatch-binding protein useful in the methods of the present invention.

As is known in the art, various hybridization conditions can be employed in the method of the present invention. Typically hybridization will proceed at slightly elevated temperatures, e.g., between about 35°C and 75°C and usually around 65°C, in a solution comprising buffer at pH between about 6 and 8 and with appropriate ionic strength (e.g., 2X SSC where 1X SSC = 0.15 M sodium chloride and 0.015 M sodium citrate, pH 7.0), protein such as bovine serum albumin, Ficoll (a trademark identifying a copolymer of sucrose and epichlorhydrin sold by Pharmacia Fine Chemicals, Piscataway, NJ) polyvinylpyrrolidone, and a denatured foreign DNA such as from calf thymus or salmon sperm. The degree of complementarity between the sample and hybridization partner strands required for hybridization to occur depends on the stringency of the conditions. The extent and specificity of hybridization is affected by the following principal conditions.
1. The purity of the nucleic acid preparation.
2. The content of G-C base pairs: G-C base pairs will exhibit greater thermal stability than A-T or A-U base pairs. Thus, hybrids with higher G-C content will be stable at higher temperatures.
3. The length of the homologous base sequence: any short sequence of bases, e.g., less than 6 bases, has a high degree of probability of recurring in many nucleic acids. Little or no specificity can be attained in hybridizations involving such short sequences. The present hybridization partner sequence will preferably be at least about 30 bases and up to about 100 bases for an oligonucleotide, or even greater for a cDNA molecule.
4. Ionic strength: the rate of reannealing increases as the ionic strength of the incubation solution increases. Thermal stability of hybrids also increases.
5. Incubation temperature: optimal reannealing occurs at a temperature about 25-30°C below the melting temperature (Tm) for a given duplex. Incubation at temperatures significantly below the optimum allows less related base sequences to hybridize.
6. Nucleic acid concentration and incubation time: Normally, to drive the reaction towards hybridization, one of the hybridizable sample nucleic acid or hybridization partner nucleic acid will be present in excess, usually 100-fold excess or greater.
7. Denaturing reagents: The presence of hydrogen bond-disrupting agents such as formamide and urea increases the stringency of hybridization.
8. Incubation time: The longer the incubation time, the more complete will be the hybridization.
9. Volume exclusion agents: The presence of these agents, as exemplified by dextran and dextran sulfate, are through to increase the effective concentrations of the hybridizing elements thereby increasing the rate of resulting hybridization.

Normally, the temperature conditions selected for hybridization will be incompatible with the binding of the mismatch-binding protein to the formed hybrids or of antibodies or other binding partners to the mismatch-binding protein. Accordingly, the mismatch-binding protein and binding partner reagent binding steps and label detection steps will proceed after completion of the hybridization step. The reaction mixture will usually be brought to a temperature in the range of from about 3°C to about 40°C and the mismatch-binding protein and additional reagent binding and detection steps then performed.

It may be expected in a particular assay using an RNA hybridization partner that the RNA will be subject to partial degradation by alkaline hydrolysis of the phosphodiester bonds or by the presence of ribonucleases (RNases). In the former case, hydrolysis can be controlled by avoiding exposure of the hybridization partner to a pH higher than about 10. RNases can be effectively inhibited by the presence of such substances as sodium dodecylsulfate, aurintricarboxylic acid, ribonucleoside vanadyl complexes, heparin diethylpyrocarbonate and proteinaceous RNase inhibitors isolated from mammalian sources.

A mismatch-binding protein bound to the hybridization partner-target polynucleotide (DNA-DNA, DNA-RNA or RNA-RNA) hybrid can be detected either directly or indirectly (see Figure 2). By direct detection is intended labeling the mismatch-binding protein with a detectable label, for example, radiolabels, fluorescent labels, conjugated enzymes, or the like, which are described in more detail below. Alternatively, as described above; the mismatch-binding protein can be produced by recombinant DNA technology as a fusion protein with a fusion partner which can easily be detected. It is important that the fusion protein, as well as a detectably labeled or conjugated mismatch-binding protein, maintain the reactivity and binding specificity of native, unmodified mismatch-binding protein. this reactivity and specificity can be ascertained by one of ordinary skill in the art by routine testing, for example, by the assay described herein.

By indirect detection is intended a method which utilizes a binding partner specific for either the mismatch-binding protein, for a reactive moiety conjugated to the mismatch-binding protein, or for a fusion protein partner of the mismatch-binding protein. When the mismatch-binding protein is MutS, a preferred binding partner is an anti-MutS antibody (or an antigen-binding fragment thereof).

Any of the antibody reagents useful in the method of the present invention may comprise whole antibodies, antibody fragments, polyfunctional antibody aggregates, or in general any substance comprising one or more specific binding sites from an antibody. The antibody may be of any immunoglobulin isotype, e.g., IgG, IgM, and so forth. Any antigen-binding fragment of any such antibody can also be employed, for instance, the Fab' or F(ab')₂ fragments. In addition, aggregates, polymers, derivatives and conjugates of immunoglobulins or their fragments can be used where appropriate.

The immunoglobulin source for an antibody reagent can be obtained in any manner such as by preparation of a conventional polyclonal antiserum or by preparation of a monoclonal or a chimeric antibody. Antiserum can be obtained by well-established techniques involving immunization of an animal, such as a mouse, rabbit, guinea pig or goat, with an appropriate immunogen, e.g., the MutS protein.

Alternatively, the binding partner may be an antibody specific for the fusion protein partner, for example, β-galactosidase, which is fused with a mismatch-binding protein. In addition, an antibody specific for haptenic group conjugated to the mismatch-binding protein molecule can be used. Another binding partner may be avidin or streptavidin, where the mismatch-binding protein has been modified by addition of biotin. In yet another embodiment, the binding partner may be a non-immunoglobulin protein which has the property of binding to an immunoglobulin molecule, for example Staphylococcal protein A or Streptococcal protein G, which are well-known in the art. The binding partner may either itself be detectably labeled or may be detected indirectly by a detectably labeled secondary binding partner, for example, a second antibody specific for the first antibody. Thus, if a rabbit-anti-mismatch-binding protein antibody serves as the first binding partner, a labeled goat-anti-rabbit immunoglobulin antibody would be a second binding partner. In another embodiment, the signal generated by presence of the fusion protein partner of the mismatch-binding protein is amplified by reaction with a specific antibody for that fusion protein (e.g., an anti-β-galactosidase antibody) which is detectably labeled. One of ordinary skill in the art can devise without undue experimentation a number of such possible first and second binding partner systems using conventional methods well-known in the art.

In yet another embodiment, the binding partner may be the MutL protein (Grilley, M. et al., J. Biol. Chem. 264:1000-1004 (1989); Modrich, P., 1989, supra; Lahue et al., 1989, supra) obtained from E. coli, S. typhimurium, or any other source, or a functional derivative thereof. A preferred functional derivative is a fusion protein between MutL and a second fusion partner protein which can be readily detected, such as β-galactosidase. The purified MutL protein which has a molecular weight of 90 kDa is known to bind selectively to the complex between MutS and a nucleic acid heteroduplex in the presence of ATP. MutL does not bind directly to a mismatch-containing heteroduplex nor to free MutS. The MutL protein has been cloned and expressed in bacteria (Pang et al., supra). Thus, for use according to the present invention, the MutL protein or functional derivative, preferably in detectably labeled form, is added to the mutation detection assay in the presence of ATP. The bound MutL is then detected. Alternatively, unlabeled MutL may be used as the first binding partner, followed by a labeled second binding partner which binds to MutL, or by an unlabeled second binding partner followed by a detectably labeled third binding partner, as discussed above. As described above for MutS binding partners, a second binding partner for MutL may be an antibody specific for MutL, an antibody specific for a hapten conjugated to MutL, avidin or streptavidin where biotin is conjugated to MutL, an antibody for the fusion partner protein of MutL, and the like.

Where the first or second binding partner described above is an antibody, detection may be accomplished using any of a variety of conventional immunoassays, including enzyme immunoassays (EIA) (Voller, A., Diagnostic Horizons 2:1-7, 1978, Microbiological Associates Quarterly Publication, Walkersville, MD; Voller, A. et al., J. Clin. Pathol. 31:507-520 (1978); U.S. Reissue Pat. No. 31,006; UK Patent 2,019,408; Butler, J.E., Meth. Enzymol. 73:482-523 (1981); Maggio, E. (ed.), Enzyme Immunoassay, CRC Press, Boca Raton, FL, 1980) or radioimmunoassays (RIA) (Weintraub, B., Principles of Radioimmunoassays, Seventh Training Course on Radioligand Assay Techniques, The Endocrine Society, March 1986, pp. 1-5, 46-49 and 68-78).

In a preferred embodiment, a MutS-specific antibody or antibody fragment is detectably labeled by linking the same to an enzyme and use it in an EIA, or enzyme-linked immunosorbent assay (ELISA). This enzyme, in turn, when later exposed to a substrate in such a manner as to produce a chemical moiety which can be detected, for example, by spectrophotometric, fluorometric or, most preferably, by visual means. Preferably, the substrate is a chromogenic substrate which generates a reaction product visible to the naked eye.

Enzymes which can be used to detectably label the binding partner for the mismatch-binding protein, such as MutS-specific antibody, include, but are not limited to, alkaline phosphatase, horseradish peroxidase, glucose-6-phosphate dehydrogenase, staphylococcal nuclease, delta-V-steroid isomerase, yeast alcohol dehydrogenase, alpha-glycerophosphate dehydrogenase, triose phosphate isomerase, asparaginase, ribonuclease, urease, catalase, glucose-6-phosphate dehydrogenase, glucoamylase and acetylcholinesterase.

By radioactively labeling the binding partner, for example, the MutS-specific antibody, it is possible to detect MutS bound to a solid support through the use of a radioimmunoassay (RIA). The radioactive isotope can be detected by such means as the use of a gamma counter or a scintillation counter or by autoradiography. Isotopes which are particularly useful for the purpose of the present invention are: ³H, ¹³¹I, ³⁵S, ¹⁴C, and preferably ¹²⁵I.

It is also possible to label the first or second binding partner with a fluorescent compound. When the fluorescently labeled antibody is exposed to light of the proper wave length, its presence can then be detected due to fluorescence. Among the most commonly used fluorescent labelling compounds are fluorescein isothiocyanate, rhodamine, phycoerythrin, phycocyanin, allophycocyanin, o-phthaldehyde and fluorescamine.

The first or second binding partner also can be detectably labeled by coupling it to a chemiluminescent compound. The presence of the chemiluminescent-tagged antibody is then determined by detecting the presence of luminescence that arises during the course of a chemical reaction. Examples of particularly useful chemiluminescent labeling compounds are luminol, isoluminol, theromatic acridinium ester, imidazole, acridinium salt and oxalate ester.

Likewise, a bioluminescent compound may be used to label the first or second binding partner. Bioluminescence is a type of chemiluminescence found in biological systems in which a catalytic protein increases the efficiency of the chemiluminescent reaction. The presence of a bioluminescent protein is determined by detecting the presence of luminescence. Important bioluminescent compounds for purposes of labeling are luciferin, luciferase and aequorin.

The test sample to be assayed can be in any medium of interest, and will generally sample of medical, veterinary, environmental, nutritional, or industrial significance. Human and animal specimens and body fluids particularly can be assayed by the present method, providing that they contain cells from which nucleic acids can be prepared. Preferred sources include blood, sperm, other tissue, milk, urine, cerebrospinal fluid, sputum, fecal matter, lung aspirates, throat swabs, genital swabs and exudates, rectal swabs, and nasopharyngeal aspirates.

Where the sample contains principally double stranded nucleic acids, such as the DNA naturally occurring in cells, the sample will first be treated to release the nucleic acid from the cells, which can be achieved by mechanical disruption (such as freeze/thaw, abrasion, sonication), physical/chemical disruption, such as treatment with detergents (e.g., Triton, Tween, or sodium dodecylsulfate), osmotic shock, heat, or enzymatic lysis (lysozyme, proteinase K, pepsin, etc.)

According to the method of the present invention, the release dsDNA may be subjected to cleavage by restriction endonuclease enzymes to form fragments of the appropriate length for the assay, and which contain the desired sequence. The choice of restriction enzymes depends on the desired target sequence and the presence of appropriate restriction enzyme recognition sites on either side of that sequence. One of ordinary skill in the art will be able to identify the site and select the enzymes without undue experimentation. See, for example, Sambrook, J. et al., supra.

Following restriction enzyme digestion, denaturation of nucleic acids is preferably accomplished by heating in boiling water or alkali treatment (e.g., 0.1 N sodium hydroxide). The resulting test medium will contain nucleic acids in single stranded form which can then be assayed according to the method of the present invention.

The present invention also provides methods and kits for detecting particular mutations in humans. Table 1, below, provides a non-limiting list of human mutations which can be detected using the methods of the present invention. See, also, Cooper, D.N. et al., Hum. Genet. 85:55-74 (1990).

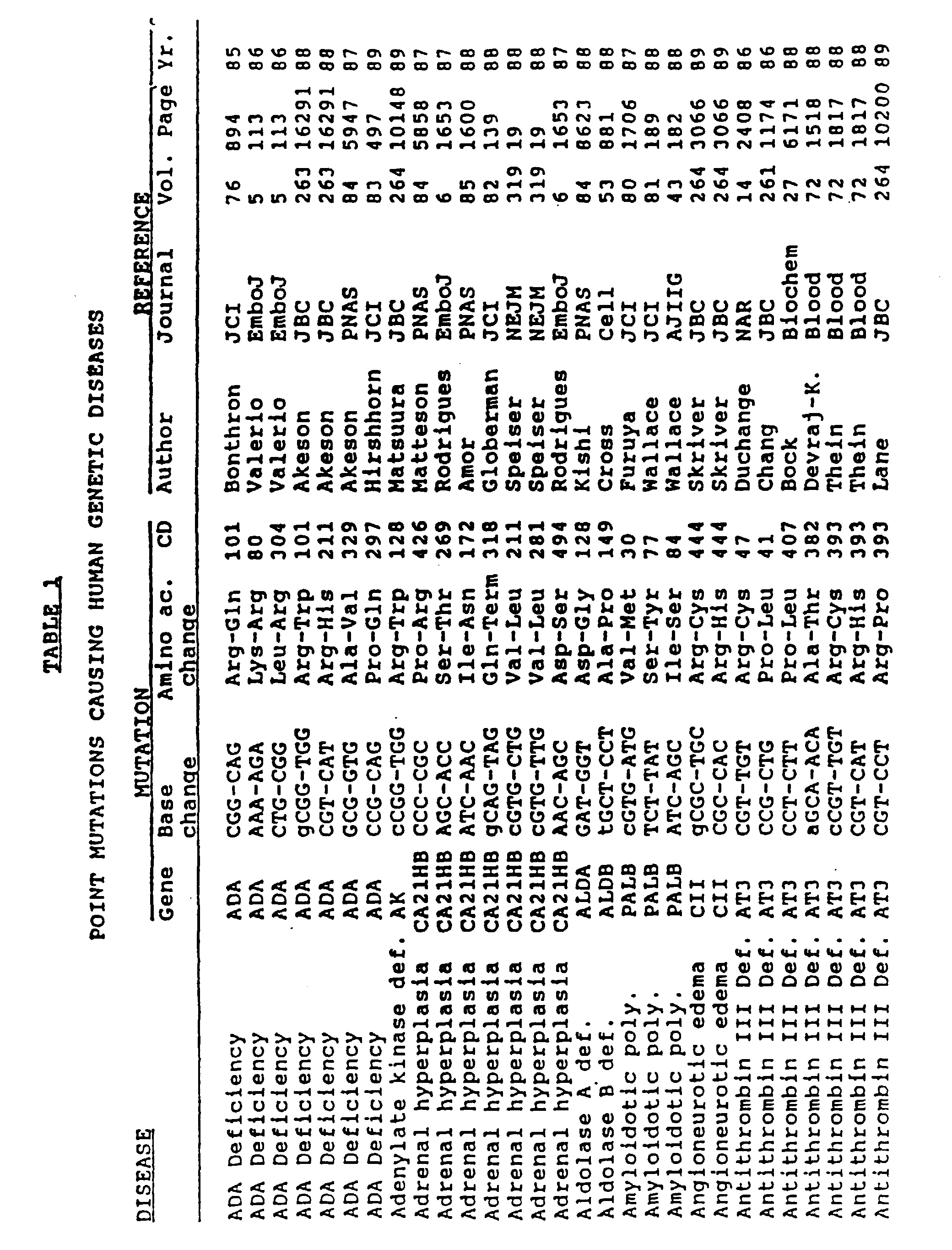

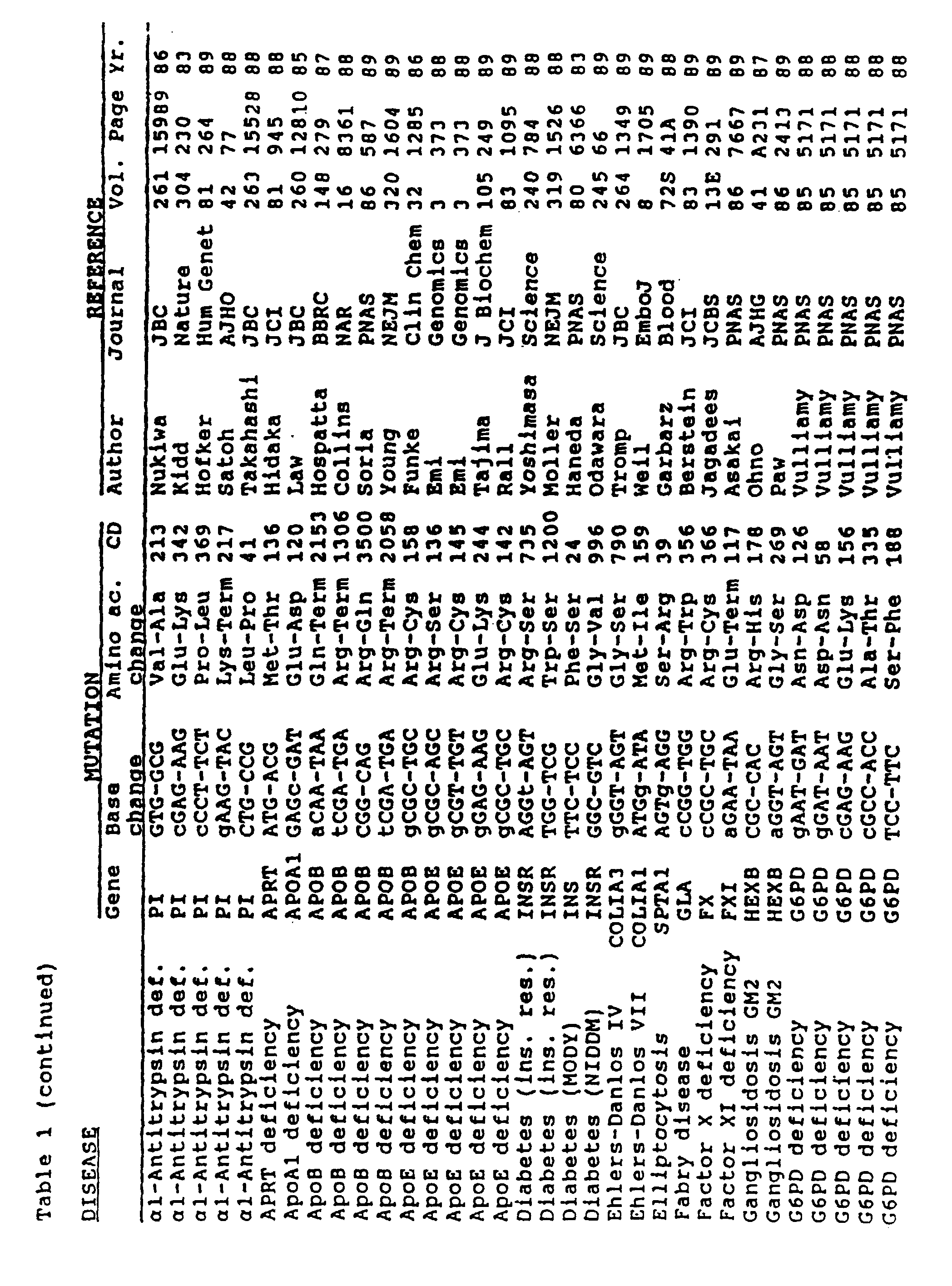

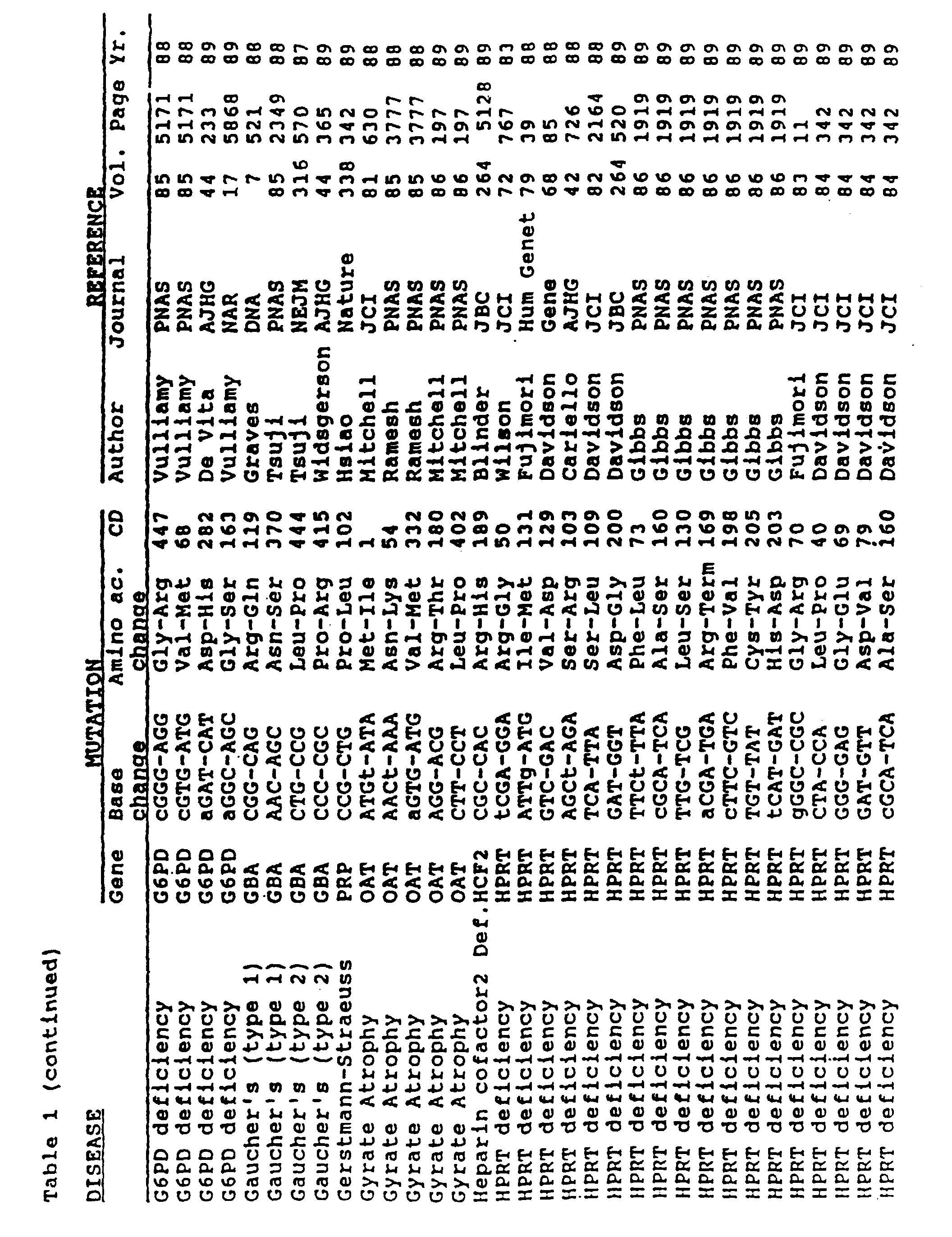

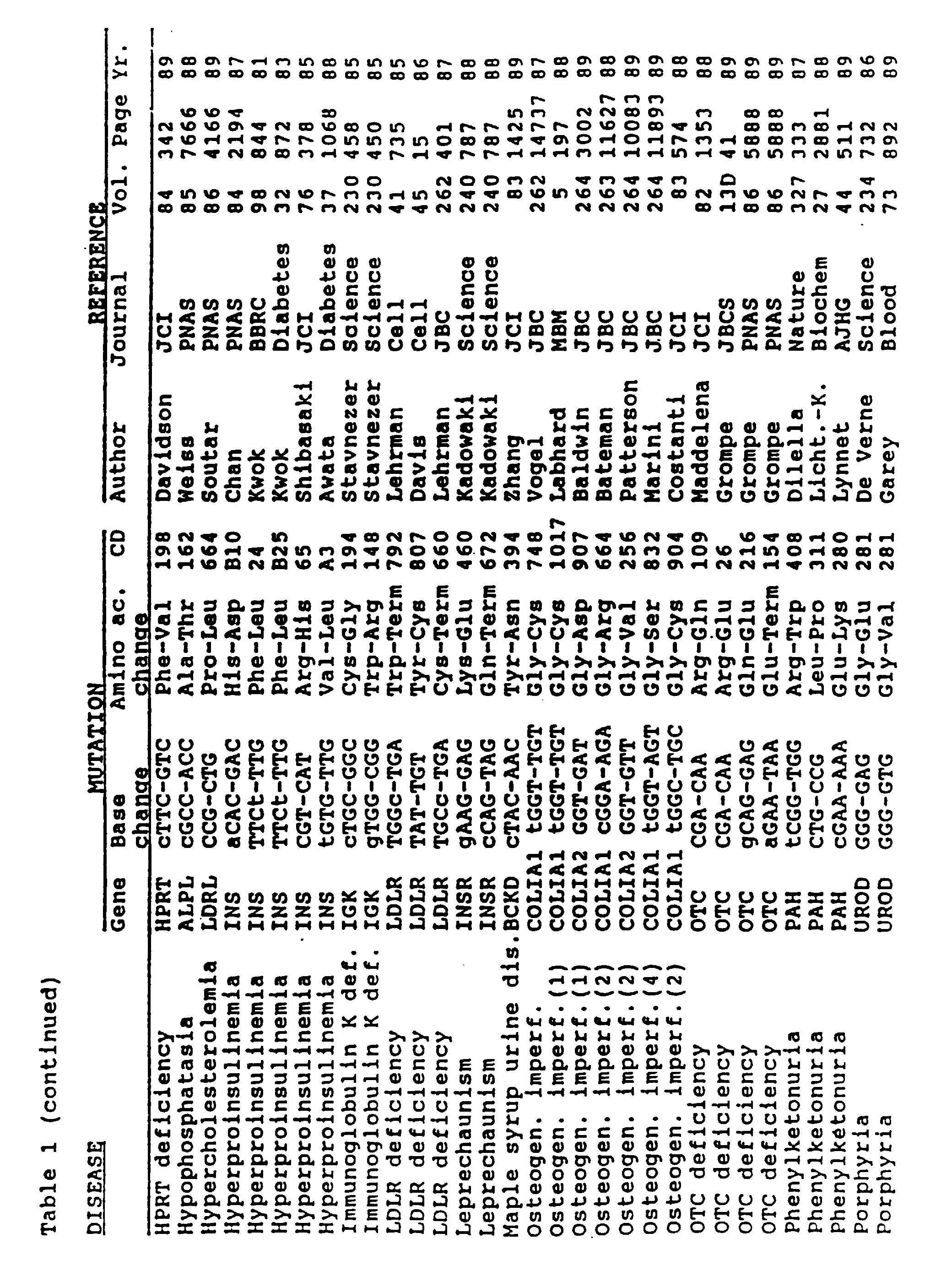

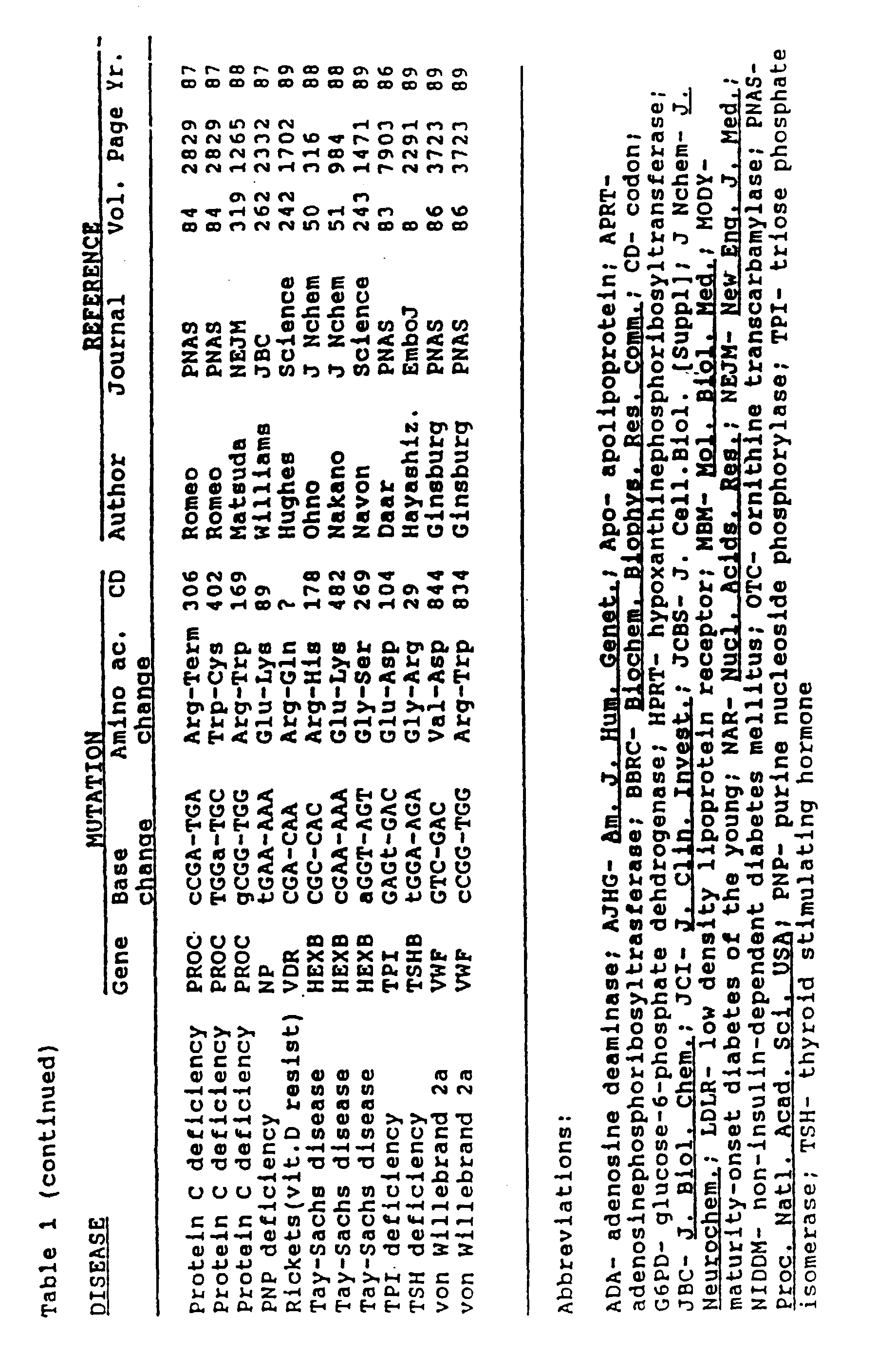

The present invention is also directed to a kit or reagent system useful for practicing the method described above. Such a kit will contain a reagent combination comprising the essential elements required to conduct an assay according to the methods disclosed herein. The reagent system is presented in a commercially packaged form, as a composition or admixture where the compatibility of the reagents will allow, in a test device configuration, or more typically as a test kit, i.e., a packaged combination of one or more containers, devices, or the like holding the necessary reagents, and usually including written instructions for the performance of assays. The kit of the present invention may include any configurations and compositions for performing the various assay formats described herein.

In all cases, the reagent system will comprise (1) an immobilizable or immobilized hybridization partner as described herein, and (2) a mismatch-binding protein or functional derivative. The mismatch-binding protein may optionally be detectably labeled. The kit may optionally contain a first binding partner for the mismatch-binding protein, e.g., an anti-MutS antibody, a MutL protein or functional derivative, and any additional binding partners or detectable label reagents. A kit according to the present invention can additionally include ancillary chemicals such as the components of the hybridization solution, a denaturation agent capable of converting double stranded nucleic acids in a test sample into single stranded form, or restriction enzymes for producing fragments of the sample nucleic acid.

Having now generally described the invention, the same will be more readily understood through reference to the following examples which are provided by way of illustration, and are not intended to be limiting of the present invention.

### EXAMPLE 1

### ASSAY OF A SAMPLE FROM BLOOD FOR A MUTANT DNA SEQUENCE

The assay format is described generally in Figure 3. Sample DNA is obtained from blood cells being tested for the presence of a particular mutation, which differs from wild-type by a single-base pair or by an addition or deletion of one or a few base pairs. The DNA is then denatured at which point it is ready to be assayed.

A test DNA hybridization partner of about 30 nucleotides is constructed to have a sequence corresponding to the wild-type gene sequence around the mutation of interest. As a positive control for a mismatch, the point mutation of the sickle cell hemoglobin is utilized. An oligonucleotide having the mutant sequence (exon 10) is produced. As a negative control for the assay, an oligonucleotide known to be of the wild-type sequence, the wild-type human hemoglobin sequence of exon 10 is produced.

The hybridization partner DNA is immobilized on nitrocellulose filters as follows:
(a) Test hybridization partner DNA in the form of a vertical line of a "plus" sign.
(b) Positive control DNA is immobilized on the filter in the form of a horizontal member of the same "plus" sign.
(c) Negative control DNA is applied to the filter in the form of a dot near the "plus" sign.

The filters are treated to fix the oligonucleotides and to block all unreacted sites to prevent non-specific binding of DNA and proteins during the assay, using conventional methods (Sambrook et al., supra).

The sample DNA is added in a concentration between 10 and 100 µg/ml and the hybridization reaction is allowed to proceed at 45-55°C in 0.1-0.3 M NaCl, resulting in the appropriate restriction fragments binding to the three oligonucleotide fragments.

The filter is washed and MutS is added to the filter and allowed to bind, and the filter is again washed.

Polyclonal rabbit anti-MutS antibodies are added to the filter, allowed to bind, and unbound antibodies washed off.

A horseradish peroxidase-conjugated goat anti-rabbit immunoglobulin antibody is then added, allowed to bind, and unbound antibody washed off.

A chromogenic substrate for horseradish peroxidase is added and the color reaction allowed to develop until a colored line (or plus sign) appears, at which time the reaction is stopped.

The results will appear as follows:
A. For a sample lacking the point mutation in the DNA (a negative):
   (1) The dot (negative control hybridization partner) is not visible, indicating that the assay was not a false positive.
   (2) Only the horizontal line is visible. Since the subject presumably has a normal Hb gene, and the positive control oligonucleotide hybridization partner had the mutant sequence, a reaction has occurred.
   (3) Thus a colored "minus" sign appears on the filter, indicating a negative result in the test for mutant DNA in the sample.
B. For a sample having the point mutation in the DNA (a positive):
   (1) The dot (negative control hybridization partner) is not visible, indicating that the assay was not a false positive.
   (2) A "plus sign" is visible. The horizontal line is visible, since the subject presumably has a normal Hb gene, and the positive control oligonucleotide hybridization partner had the mutant sequence, resulting in a reaction. Because the DNA from this sample has a point mutation in the target DNA sequence, the vertical line of the "plus sign" yields a colored reaction product.

### EXAMPLE II

### ASSAY FOR A MUTANT DNA SEQUENCE USING A MutL BINDING PARTNER

The general assay format is similar to that described in Example I. Sample DNA is obtained from blood cells being tested for the presence of a particular mutation, which differs from wild-type by a single-base pair or by an addition or deletion of one or a few base pairs. The DNA is then denatured at which point it is ready to be assayed.

A test DNA hybridization partner of about 30 nucleotides is constructed to have a sequence corresponding to the wild-type gene sequence around the mutation of interest. As a positive control for a mismatch, the point mutation of the sickle cell hemoglobin mutation is utilized. An oligonucleotide having the mutant sequence (exon 10) is produced. As a negative control for the assay, an oligonucleotide known to be of the wild-type sequence, the wild-type human hemoglobin sequence of exon 10 is produced.

The hybridization partner DNA is immobilized on nitrocellulose filters as follows:
(a) Test hybridization partner DNA in the form of a vertical line of a "plus" sign.
(b) Positive control DNA is immobilized on the filter in the form of a horizontal member of the same "plus" sign.
(c) Negative control DNA is applied to the filter in the form of a dot near the "plus" sign.

The filters are treated to fix the oligonucleotides and to block all unreacted sites to prevent non-specific binding of DNA and proteins during the assay, using conventional methods (Sambrook et al., supra).

The sample DNA is added in a concentration between 10 and 100 µg/ml and the hybridization reaction is allowed to proceed at 45-55°C in 0.1-0.3 M NaCl, resulting in the appropriate restriction fragments binding to the three oligonucleotide fragments.

The filter is washed and MutS is added to the filter and allowed to bind, and the filter is again washed.

A MutL-β-galactosidase fusion protein is added as a first binding partner and allowed to bind, and unbound material washed off.

The second binding partner, a polyclonal rabbit anti-β-galactosidase antiserum is added to the filter, allowed to bind, and unbound antibodies washed off.

The third binding partner, a horseradish peroxidase-conjugated goat anti-rabbit immunoglobulin antibody is then added, allowed to bind, and unbound antibody washed off.

A chromogenic substrate for horseradish peroxidase is added and the color reaction allowed to develop until a colored line (or plus sign) appears, at which time the reaction is stopped.

The results will appear as follows:
A. For a sample lacking the point mutation in the DNA (a negative):
   (1) the dot (negative control hybridization partner) is not visible, indicating that the assay was not a false positive.
   (2) Only the horizontal line is visible. Since the subject presumably has a normal Hb gene, and the positive control oligonucleotide hybridization partner had the mutant sequence, a reaction has occurred.
   (3) Thus a colored "minus" sign appears on the filter, indicating a negative result in the test for mutant DNA in the sample.
B. For a sample having the point mutation in the DNA (a positive):
   (1) the dot (negative control hybridization partner) is not visible, indicating that the assay was not a false positive.
   (2) A "plus" sign is visible. Because the DNA from this sample has a point mutation in the target DNA sequence, the vertical line of the "plus sign" yields a colored reaction product.

### EXAMPLE III

### DETECTION OF A MUTATED p53 ONCOGENE IN A TISSUE SAMPLE

The p53 gene appears to be a tumor suppressor gene (Levine, A. et al., Nature 351:453-455 (1991)) wherein mutation at numerous sites can result in expression of a tumor, in particular colorectal carcinoma. See, also, for example, Kinzler, K. W. et al., Science 251:1366-1370 (1991); Kern, S. E. et al., Oncogene 6:131-136 (1991); Vogelstein, B., Nature 348:681-682 (1990); Baker, S. J. et al., Science 249:912-915 (1990).

Human DNA is isolated from tumor tissue. The isolated DNA is sheared under standard conditions. The DNA is then denatured at which point it is ready to be assayed.

A cDNA molecule corresponding to the p53 gene is produced by standard methods (Sambrook et al., supra) and serves as the hybridization partner. Regions harboring known mutations may be scattered throughout the p53 sequence.

The hybridization partner DNA is immobilized on nitrocellulose filters as follows (see Figure 3):
(a) The test hybridization partner (p53 cDNA) is spotted in the form of a vertical line of a "plus" sign.
(b) Positive control DNA, is immobilized on the filter in the form of a horizontal member of the same "plus" sign.
(c) Negative control DNA is applied to the filter in the form of a dot near the "plus" sign.

The filters are treated to fix the hybridization partner poly- or oligonucleotides and to block all unreacted sites to prevent non-specific binding of DNA and proteins during the assay.

The sample DNA is added and the reaction is incubated under hybridizing conditions (as described above), except that the hybridization temperature is 55-60°C, resulting in the appropriate DNA fragments binding to the three hybridization partner poly- or oligonucleotides.

The filter is washed and MutS is added to the filter and allowed to bind, and the filter is again washed.

Polyclonal rabbit anti-MutS antibodies are added to the filter, allowed to bind, and unbound antibody washed off.

A horseradish peroxidase-conjugated goat anti-rabbit immunoglobulin antibody is then added, allowed to bind, and unbound antibody washed off.

A chromogenic substrate for horseradish peroxidase is added and the color reaction allowed to develop until a colored "minus" or "plus" sign appears, at which time the reaction is stopped.

The results will appear as follows:
A. For a sample lacking the mutant p53 oncogene:
   (1) The dot (negative control hybridization partner) is not visible, indicating that the assay was not a false positive.
   (2) only the horizontal line is visible. Since the subject presumably has a non-mutated sequence complementary to the positive control DNA, and the positive control oligonucleotide hybridization partner has a mutant sequence, a reaction occurs.
   (3) Thus a colored "minus" sign appears on the filter, indicating a negative result in the test for a mutant p53 oncogene in the sample DNA.
B. For a sample having a mutant p53 oncogene:
   (1) The dot (negative control hybridization partner) is not visible, indicating that the assay was not a false positive.
   (2) A "plus" sign is visible. The horizontal line is visible, since the subject presumably has a normal Hb gene, and the positive control oligonucleotide hybridization partner had the mutant sequence, resulting in a reaction. Because the DNA from this sample has at least one point mutation in the target DNA sequence, the vertical line of the "plus sign" yields a colored reaction product.

### EXAMPLE IV

### DETECTION OF EXPRESSION OF A MUTATED p53 ONCOGENE

Human mRNA is isolated from tumor tissue by conventional methods (see, Sambrook et al., supra). A cDNA molecule corresponding to the p53 gene is produced by standard methods (Sambrook et al., supra) and serves as the hybridization partner. The assay format is described generally in Figure 3.

The hybridization partner DNA is immobilized on nitrocellulose filters as follows:
(a) The test hybridization partner (p53 DNA) is spotted in the form of a vertical line of a "plus" sign.
(b) Positive control DNA comprises an oligonucleotide sequence corresponding to a gene known to be expressed in the tumor tissue, which has had one base modified. This DNA is immobilized on the filter in the form of a horizontal member of the same "plus" sign.
(c) Negative control DNA, comprising the wild-type sequence of the oligonucleotide used as the positive control, is applied to the filter in the form of a dot near the "plus" sign.

The filters are treated to fix the hybridization partner poly- or oligonucleotides and to block all unreacted sites to prevent non-specific binding of nucleic acid and proteins during the assay.

The sample mRNA is added and the reaction is incubated under hybridizing conditions, resulting in mRNA binding to the three hybridization partner poly- or oligonucleotides.

The filter is washed and MutS is added to the filter and allowed to bind, and the filter is again washed.

Polyclonal rabbit anti-MutS antibodies are added to the filter, allowed to bind, and unbound antibody washed off.

A horseradish peroxidase-conjugated goat anti-rabbit immunoglobulin antibody is then added, allowed to bind, and unbound antibody washed of.

A chromogenic substrate for horseradish peroxidase is added and the color reaction allowed to develop until a colored "minus" or "plus" sign appears, at which time the reaction is stopped.

The results will appear as follows:
A. For a sample expressing the wild-type p53 gene:
   (1) The dot (negative control hybridization partner) is not visible, indicating that the assay was not a false positive.
   (2) Only the horizontal line is visible. Thus a colored "minus" sign appears on the filter, indicating a negative result in the test for expression of a mutant p53 oncogene in the sample mRNA.
B. For a sample expressing a mutant p53 gene:
   (1) The dot (negative control hybridization partner) is not visible, indicating that the assay was not a false positive.
   (2) A "plus sign" is visible. The horizontal line is visible, since the subject presumable has a non-mutated sequence complementary to the positive control DNA, and the positive control oligonucleotide hybridization partner has a mutant sequence, a reaction occurs. Because the mRNA from this sample has at least one point mutation in the target sequence, the vertical line of the "plus sign" yields a colored reaction product.

### EXAMPLE V

### MISMATCH DETECTION BY mutS PROTEIN

### MATERIALS AND METHODS

Cells. 1.2L (lambda cI857 N^{†} ΔH kil⁻) was made a mutS overproducer strain by the introduction of the mutS expressing plasmid pMS312 (Su and Modrich, Proc. Nat. Acad. Sci. USA. 83:5057, 1986).

Purification of mutS. The mutS purification scheme is a modification of that described by Su and Modrich (ibid.). 1.2L (pMS312) cells were grown at 30°C in 8 liters of 869 medium (10 g/l Bacto-Tryptone (Difco), 5 g/l yeast extract (Difco), 5 g/l NaCl) containing 50 µg/ml ampicillin to an OD₅₉₀ of 1.3-1.8. Cultures were diluted 1:2 in 869 medium prewarmed to 60°C and incubated at 42°C for 5 hours. Cultures were chilled, the cells harvested by centrifugation and the cell past stored at -70°C. Cell past (15-29 gm) was thawed on ice and the cells resuspended in an equal volume of buffer A (20mM KPO₄, pH7.4, 1mM EDTA, 1mM PMSF, 10 mM 2-mercaptoethanol). Cells were lysed by sonication in an ice-acetone bath. The lysate was clarified by centrifugation (40,000g; 30 minutes). The supernatant was treated with 1/4 volume 25% (w/v) streptomycin sulfate in buffered A and stirred for 45 minutes at 4°C. Insoluble material was removed by centrifugation (40,000g; 30 minutes). Ammonium sulfate (0.2g per ml of supernatant) was added over a 15 minute period and the suspension stirred for an additional 40 minutes at 4°C. The precipitate was collected by centrifugation (40,000g; 30 minutes) and resuspended in 10ml of buffer A. 1ml fractions were diluted 1:10 in buffer B (20mM KPO₄, pH 7.4, 0.1mm EDTA, 1mm PMSF, forum 2-mercaptoethanol) containing 0.025M kCL and applied immediately at 2 ml/min to a 1.5 x 30cm Heparin-Separose CL6B (Pharmacia) column. Column was washed with 100ml buffer B containing 0.1M KCl and eluted with a 300 ml linear gradient of KCl (0.1-0.5M) in buffer B. 100 3ml fractions were collected. Samples were run on 4-20% SDS-PAGE gels under reducing conditions and those fractions containing >95% mutS protein (97 kDa) were pooled. Glycerol was added to the pooled material to a final concentration of 20% and aliquots were stored at - 70°C.

Oligonucleotides. Oligonucleotides were obtained from Biopolymer Labs. The sequence of the oligonucleotides was taken from the 30 base region surrounding the site of sickle cell mutation in the human β-globin gene. In order to allow oligonucleotides to be immobilized on nitrocellulose filters, selected oligonucleotides were prepared with a 5' 25mer poly-C "tail" and a 5' amino-modifier. The sequences used in these experiments are presented in Table 2. (Note: The actual sickle cell mutation is an A:T → T:A transversion.) Oligonucleotides were annealed in various combinations to produce G:T, A:C and G:G mismatches, a heteroduplex with one unpaired base (such as would be formed by annealing an oligonucleotide with a wild type sequence to one with a one base addition or deletion mutation) and two different homoduplexes, i.e., perfectly complementary duplexes. Annealing conditions are described below.

Anti-mutS antibodies. Polyclonal anti-mutS antibodies were prepared from serum obtained by injecting New Zealand White rabbits (Pel Freez) with purified mutS protein. Antibodies were partially purified by protein G - sepharose (Pharmacia) column chromatography. In dot blot analysis, 10µg/ml of anti-mutS antibody was found to detect 1ng purified mutS protein.

### RESULTS

### Detection of mismatches in heteroduplexes annealed in solution.

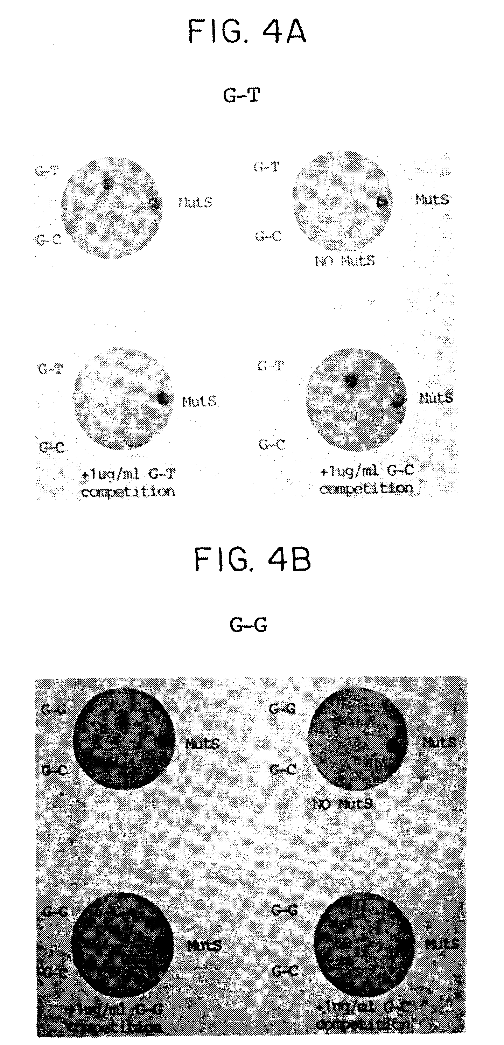

The data presented in Figure 4 indicates that G:T and G:G containing heteroduplexes can be detected by mutS protein. The detection depends on mutS and can be competed away with heteroduplex, but not homoduplex, DNA. Similar results were obtained with A:C mismatches and heteroduplexes with one unpaired base (data not shown).

### Annealing to immobilized oligonucleotides.

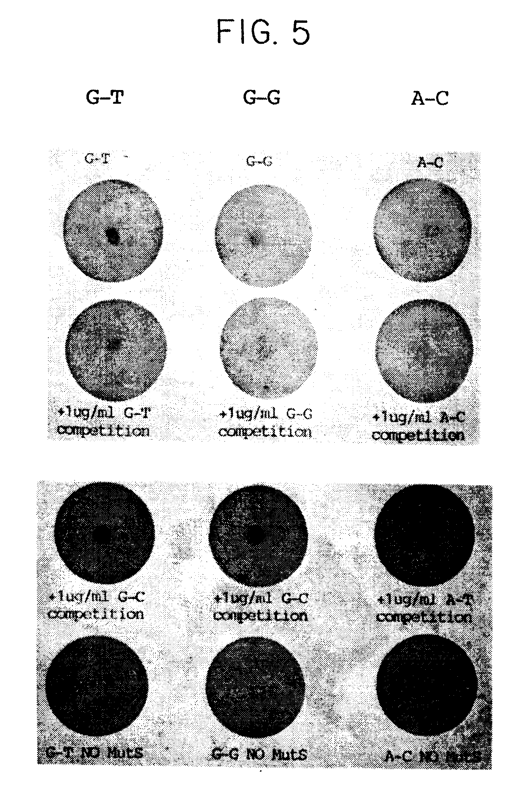

"Tailed" oligonucleotides were spotted on filters and 30mer oligonucleotides annealed to them. The results (Figure 5) are identical to those obtained with heteroduplexes prepared by annealing in solution (Figure 4). Homoduplexes (formed by using 30mer oligonucleotides exactly complementary to the 30mer portion of the immobilized oligonucleotides) were not detected.

### Annealing in the presence of competing oligonucleotides.

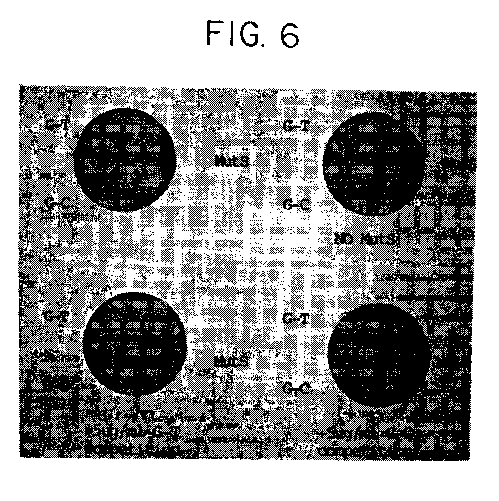

Homoduplexes were prepared by annealing complementary 30mers. "Tailed" oligonucleotides were then added, the homoduplexes denatured and the mixture reannealed. Samples were spotted on filters. Figure 6 shows that G:T mismatches are detected under such conditions, indicating that the "tailed" oligonucleotides effectively compete with 30mers for complementary oligonucleotides. Such conditions parallel those that could be used in a mutation detection assay. In such an assay, the sample DNA could be cut by restriction enzymes (for genomic DNA) or prepared by PCR. In either case the DNA would be double stranded and, therefore, contain sequences identical to and capable of competing with the tailed oligonucleotides used as probes.

Results presented in Figure 4 were obtained from experiments carried out as follows:
Heteroduplexes and homoduplexes were prepared by annealing equal amounts of oligonucleotides. Since the "tailed" oligonucleotides are 55 bases long, there was nearly a 2-fold excess of 30mers, increasing the likelihood that all "tailed" oligonucleotides would become double stranded. 5µg (in 50µl) of each oligonucleotide in TNE (10mM Tris, pH 8.0, 0.1M NaCl, 1mM EDTA) were mixed, heated to 70°C for 10 minutes, cooled to room temperature for 30 minutes and chilled on ice. Annealed molecules (1µg in 2µl) were spotted on 25mm nitrocellulose disks (0.45 micron pore size, Scleicher and Schuell). 10ng of purified mutS was spotted on the filters as a positive control for the primary (anti-mutS) and secondary antibodies and the alkaline phosphatase development system. Filters were air dried and transferred to 6 well tissue culture plates (Falcon) containing 2ml reaction buffer (20mM Tris, pH 7.4, 0.01mM EDTA, 0.5mM MgCl₂, 0.01mM DTT) + 3% (w/v) non-fat dry milk and incubated overnight at 4°C with gentle shaking. Filters were washed with cold reaction buffer (4x2ml) and incubated (30 minutes at 4°C) with single strand binding protein (Promega) solution (1ml at 100µg/ml in reaction buffer). Filters were washed with cold reaction buffer (4x2ml) and incubated (30 minutes at 4°C) with (i) 1ml reaction buffer, (ii) 1ml reaction buffer + 10µg/ml mutS or (iii) 1ml reaction buffer + 10µg/ml mutS + 1µg/ml homoduplex or heteroduplex 30mers, annealed as described above. Filters were washed with cold reaction buffer (4x2ml) and incubated (2 hours at 4°C) with anti-mutS antibodies (2ml at 50µg/ml in reaction buffer + 3% non-fat dry milk). Filters were washed with cold reaction buffer (4x2ml) and incubated (1 hour at 4°C) with 2ml of a 1:1000 dilution (in reaction buffer +3% non-fat dry milk of goat anti-rabbit IgC conjugated to alkaline phosphatase (Bio-Rad). Filters were washed with cold reaction buffer (4x2ml) and incubated (10-20 minutes at room temperature) with 2ml fresh development buffer (100mM Tris, pH 9.5, 100mM NaCl, 5mM MgCl₂, .165mg/ml Bromochloroindoyl phosphate (diluted 1:100 from stock solution in dimethylformamide), 0.33 mg/ml Nitro-blue tetrazolium (diluted 1:100 from stock solution in dimethylformamide)). Filters were washed several times with water to stop the reaction.

Results presented in Figure 5 were obtained from experiments carried out as follows:
1µg (2µ) of "tailed" oligonucleotides were spotted on nitrocellulose filters. The filters were air dried and transferred to 6 well plates containing 2ml of TNE. 2µg (4µl) of 30mer oligonucleotides were added. The plates were floated in a 70°C water bath for 10 minutes, incubated at room temperature for 30 minutes and placed on ice. Filters were then treated as described above in relation to results presented in Figure 4.

Results presented in Figure 6 were obtained from experiments carried out as follows:
Equal amounts of 30mer oligonucleotides were annealed as described in the legend to Figure 4. An equal number of "tailed" oligonucleotides were added and the mixtures heated to 85°C for 10 minutes followed by incubation at room temperature for 30 minutes. All subsequent steps were as described in the legend to Figure 4.

It should be pointed out that recA protein can be used in lieu of the single-strand DNA binding protein, in order to facilitate annealing.

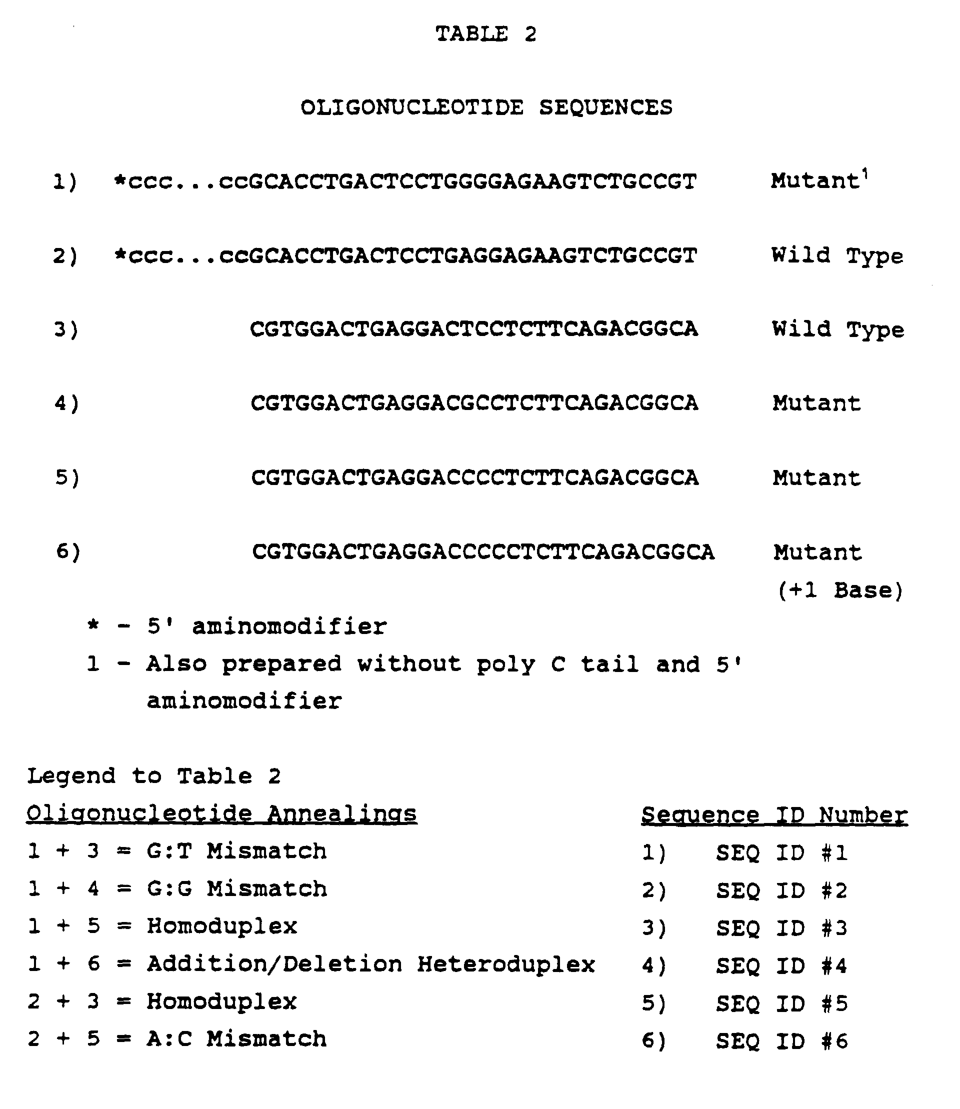

## Claims

1. A method for detecting a mutation from a non-mutated sequence of a single stranded target polynucleotide in a sample, comprising:
(a) incubating the sample with a single-stranded polynucleotide hybridization partner comprising at least one single stranded base sequence which is complementary to the non-mutated sequence of the target polynucleotide, under conditions suitable to cause hybridization of said hybridization partner to any mutated or non-mutated sequences of said target polynucleotide which may be present in the sample to form a hybrid;
(b) contacting the hybrid formed in step (a) with a mismatch-binding protein; and
(c) detecting the presence of any mismatch-binding protein bound to said hybrid,
whereby the detection of the presence of the mismatch-binding protein is indicative of the presence of a mutation in the sequence of the polynucleotide of the sample.

2. A method according to claim 1 wherein said polynucleotide hybridization partner is DNA, e.g. cDNA, and/or said target polynucleotide is DNA.

3. A method according to claim 1 wherein said mismatch-binding protein is the MutS protein or a functional derivative thereof.

4. A method according to claim 1 wherein said sample is a biological specimen or fluid which has been subjected to conditions sufficient to release and denature any nucleic acid present therein, and optionally said released nucleic acid is subjected to a step of restriction endonuclease cleavage prior to said denaturation.

5. A method according to claim 1 wherein said polynucleotide hybridization partner is immobilized on a solid support prior to said incubating of step (a), and for instance said solid support is a nitrocellulose membrane.

6. A method according to claim 1 wherein said detection step comprises addition of a detectably labelled first binding partner capable of binding to said mismatch-binding protein, and e.g. said first binding partner is an antibody specific for said mismatch-binding protein.

7. A method according to claim 1 wherein said detection step comprises addition of a first binding partner which is not detectably labelled, said first binding partner being capable of binding to said mismatch-binding protein, and a second binding partner capable of binding to said first binding partner and detecting the presence of said second binding partner, and e.g. said second binding partner is an antibody specific for said first binding partner.

8. A method according to claim 3 wherein said detection step comprises addition of a first binding partner and (a) said first binding partner is a MutL protein or a functional derivative thereof, and is capable of being detected, or (b) said first binding partner is a fusion protein between MutL and a second protein or peptide capable of being detected, said second protein, e.g. being beta-galactosidase.

9. A method according to claim 1 wherein said mismatch-binding protein of step (b) is a fusion protein fused with a second protein or peptide capable of being detected, and e.g. said detection step comprises addition of a first binding partner capable of binding to said second protein or peptide and detecting the presence of said first binding partner, and e.g. said second protein is beta-galactosidase.

10. A method according to claim 9 wherein said second protein is an enzyme capable of catalyzing the generation of a colored reaction product from a chromogenic enzyme substrate and said detection step comprises providing said chromogenic substrate and visualizing said color reaction product.

11. A method for detecting a mutation from a non-mutated sequence of a single stranded target mammalian polynucleotide in a sample, comprising:
(a) incubating the sample with a single-stranded polynucleotide hybridization partner comprising at least one single stranded base sequence which is complementary to the non-mutated sequence of the target mammalian polynucleotide, under conditions suitable to cause hybridization of said hybridization partner to any mutated or non-mutated sequences of said mammalian target polynucleotide which may be present in the sample to form a hybrid;
(b) contacting the hybrid formed in step (a) with a mismatch-binding protein; and
(c) detecting any binding of said mismatch-binding protein and said hybrid,
whereby the detection of the binding of the mismatch-binding protein is indicative of the presence of a mutation in the sequence of the mammalian polynucleotide of the sample.

12. A kit useful for detecting a mutation from a non-mutated sequence of a target polynucleotide sequence in a sample, in a method according to any one of the preceding claims, said kit being adapted to receive therein one or more containers, said kit comprising:
(a) a first container containing a single-stranded polynucleotide hybridization partner comprising at least one single stranded base sequence which is complementary to the non-mutated sequence of the target polynucleotide;
(b) a second container containing a mismatch-binding protein; and
(c) a third container or a plurality of containers containing a reagent or reagents capable of detecting the binding of said mismatch-binding protein to said hybrid.

13. A kit according to claim 12 wherein said mismatch-binding protein is MutS or a functional derivative thereof.

14. A kit according to claim 12 wherein said reagent or reagents comprise at least a first binding partner capable of binding to the mismatch-binding protein, and e.g. said first binding partner is an antibody specific for the mismatch-binding protein.

15. A kit according to claim 12 wherein said reagent or reagents comprise a first binding partner which is not detectably labelled, said first binding partner being capable of binding to said mismatch-binding protein, and a second binding partner capable of binding to said first binding partner, and e.g. said second binding partner is an antibody specific for said first binding partner.

16. A kit according to claim 15 wherein said first binding partner is a MutL protein or a functional derivative thereof, or is a fusion protein between MutL and a second protein or peptide capable of being detected, and e.g. said second protein is beta-galactosidase.

17. A kit according to claim 16 wherein said second binding partner is capable of binding to said MutL protein or functional derivative.

18. A kit according to claim 12 wherein said hybridization partner is immobilized on a solid support such as a nitrocellulose membrane.

19. A kit according to claim 12 wherein said mismatch-binding protein is a fusion protein fused with an enzyme and said reagent comprises a chromogenic substrate for said enzyme.

## Patentansprüche

1. Ein Verfahren zur Detektion einer Mutation von einer nichtmutierten Sequenz eines einzelsträngigen Ziel-Polynucleotids in einer Probe, wobei das Verfahren umfaßt:
(a) Inkubation der Probe mit einem einzelsträngigen Polynucleotid-Hybridisierungspartner, der mindestens eine einzelsträngige Basensequenz umfaßt, die zu der nichtmutierten Sequenz des Ziel-Polynucleotids komplementär ist, unter geeigneten Bedingungen für eine herbeizuführende Hybridisierung besagten Hybridisierungspartners an beliebige mutierte oder nichtmutierte Sequenzen von besagtem Ziel-Polynucleotid, das in der Probe vorhanden sein kann, um ein Hybrid zu bilden;
(b) In-Kontakt-Bringen des in Schritt (a) gebildeten Hybrids mit einem Fehlpaarungen bindenden Protein; und
(c) Detektieren des Vorhandenseins eines beliebigen an besagtes Hybrid gebundenen Fehlpaarungen bindenden Proteins,
wonach die Detektion des Vorhandenseins des Fehlpaarungen bindenden Proteins ein Hinweis auf das Vorhandensein einer Mutation in der Sequenz des Polynucleotids der Probe ist.

2. Ein Verfahren gemäß Anspruch 1, worin besagter Polynucleotid-Hybridisierungspartner DNS, z.B. cDNS, ist und/oder besagtes Ziel-Polynucleotid DNS ist.

3. Ein Verfahren gemäß Anspruch 1, worin besagtes Fehlpaarungen bindendes Protein das MutS Protein oder ein funktionales Derivat davon ist.

4. Ein Verfahren gemäß Anspruch 1, worin besagte Probe eine biologische Probe oder Flüssigkeit ist, die hinreichenden Bedingungen ausgesetzt worden ist, um eine beliebige darin vorhandene Nucleinsäure freizusetzen und zu denaturieren, und besagte freigesetzte Nucleinsäure vor besagter Denaturierung gegebenenfalls einem Restriktionsendonuclease-Spaltungsschritt unterzogen wird.

5. Ein Verfahren gemäß Anspruch 1, worin besagter Polynucleotid-Hybridisierungspartner vor besagtem Inkubationsschritt (a) an einem festen Träger immobilisiert wird, und zum Beispiel besagter fester Träger eine Nitrocellulose-Membran ist.

6. Ein Verfahren gemäß Anspruch 1, worin besagter Detektionsschritt Zugabe eines detektierbar markierten ersten bindenden Partners umfaßt, der in der Lage ist, an besagtes Fehlpaarungen bindendes Protein zu binden, und z.B. besagter erster bindender Partner ein für besagtes Fehlpaarungen bindendes Protein spezifischer Antikörper ist.

7. Ein Verfahren gemäß Anspruch 1, worin besagter Detektionsschritt Zugabe eines ersten bindenden Partners, der nicht detektierbar markiert ist, wobei besagter erster bindender Partner in der Lage ist, an besagtes Fehlpaarungen bindendes Protein zu binden, und eines zweiten bindenden Partners umfaßt, der in der Lage ist, an besagten ersten bindenden Partner zu binden und das Vorhandensein von besagtem zweiten bindenden Partner zu detektieren, und z.B. besagter zweiter bindender Partner ein für besagten ersten bindenden Partner spezifischer Antikörper ist.

8. Ein Verfahren gemäß Anspruch 3, worin besagter Detektionsschritt Zugabe eines ersten bindenden Partners umfaßt und (a) besagter erster bindender Partner ein MutL Protein oder ein funktionales Derivat davon ist und detektierbar ist, oder (b) besagter erster bindender Partner ein Fusionsprotein aus MutL und einem zweiten Protein oder Peptid ist, das detektierbar ist, wobei besagtes zweites Protein z.B. beta-Galactosidase ist.

9. Ein Verfahren gemäß Anspruch 1, worin besagtes Fehlpaarungen bindendes Protein von Schritt (b) ein Fusionsprotein ist, das mit einem zweiten Protein oder Peptid fusioniert ist, das detektierbar ist, und z.B. besagter Detektionsschritt Zugabe eines ersten bindenden Partners umfaßt, der in der Lage ist, an besagtes zweites Protein oder Peptid zu binden und das Vorhandensein von besagtem ersten bindenden Partner zu detektieren, und z.B. besagtes zweites Protein beta-Galactosidase ist.

10. Ein Verfahren gemäß Anspruch 9, worin besagtes zweites Protein ein Enzym ist, das in der Lage ist, die Bildung eines farbigen Reaktionsprodukts aus einem chromogenen Enzymsubstrat zu katalysieren, und besagter Detektionsschritt die Bereitstellung besagten chromogenen Substrats und Visualisierung besagten farbigen Reaktionsprodukts umfaßt.

11. Ein Verfahren zur Detektion einer Mutation von einer nichtmutierten Sequenz eines einzelsträngigen Ziel-Säugerpolynucleotids in einer Probe, wobei das Verfahren umfaßt:
(a) Inkubation der Probe mit einem einzelsträngigen Polynucleotid-Hybridisierungspartner, der mindestens eine einzelsträngige Basensequenz umfaßt, die zu der nichtmutierten Sequenz des Ziel-Säugerpolynucleotids komplementär ist, unter geeigneten Bedingungen für eine herbeizuführende Hybridisierung besagten Hybridisierungspartners an beliebige mutierte oder nichtmutierte Sequenzen von besagtem Ziel-Säugerpolynucleotid, das in der Probe vorhanden sein kann, um ein Hybrid zu bilden;
(b) In-Kontakt-Bringen des in Schritt (a) gebildeten Hybrids mit einem Fehlpaarungen bindenden Protein; und
(c) Detektieren einer beliebigen Bindung von besagtem Fehlpaarungen bindenden Protein und besagtem Hybrid,
wonach die Detektion der Bindung des Fehlpaarungen bindenden Proteins ein Hinweis auf das Vorhandensein einer Mutation in der Sequenz des Säuger-Polynucleotids der Probe ist.

12. Ein Set, das zur Detektion einer Mutation von einer nichtmutierten Sequenz einer Ziel-Polynucleotidsequenz in einer Probe zweckdienlich ist, in einem Verfahren gemäß einem der vorausgehenden Ansprüche, wobei besagtes Set ausgelegt ist, einen oder mehrere Behälter zu enthalten, und besagtes Set umfaßt:
(a) einen ersten Behälter, der einen einzelsträngigen Hybridisierungspartner enthält, der mindestens eine einzelsträngige Basensequenz umfaßt, die zu der nichtmutierten Sequenz des Ziel-Polynucleotids komplementär ist;
(b) einen zweiten Behälter, der ein Fehlpaarungen bindendes Protein enthält; und
(c) einen dritten Behälter oder mehrere Behälter, die ein Reagenz oder Reagenzien enthalten, die in der Lage sind, die Bindung besagten Fehlpaarungen bindenden Proteins an besagtes Hybrid zu detektieren.

13. Ein Set gemäß Anspruch 12, worin besagtes Fehlpaarungen bindendes Protein MutS oder ein funktionales Derivat davon ist.

14. Ein Set gemäß Anspruch 12, worin besagtes Reagenz oder besagte Reagenzien mindestens einen ersten bindenden Partner umfassen, der in der Lage ist, an das Fehlpaarungen bindende Protein zu binden, und z.B. besagter erster Partner ein für das Fehlpaarungen bindende Protein spezifischer Antikörper ist.

15. Ein Set gemäß Anspruch 12, worin besagtes Reagenz oder besagte Reagenzien einen ersten bindenden Partner, der nicht detektierbar markiert ist, wobei besagter erster bindender Partner in der Lage ist, an besagtes Fehlpaarungen bindendes Protein zu binden, und einen zweiten bindenden Partner umfassen, der in der Lage ist, an den ersten bindenden Partner zu binden, und z.B. besagter zweiter bindender Partner ein für besagten ersten bindenden Partner spezifischer Antikörper ist.

16. Ein Set gemäß Anspruch 15, worin besagter erster bindender Partner ein MutL Protein oder ein funktionales Derivat davon ist, oder ein Fusionsprotein aus MutL und einem zweiten Protein oder Peptid ist, das detektierbar ist, und z.B. besagtes zweites Protein beta-Galactosidase ist.

17. Ein Set gemäß Anspruch 16, worin besagtes zweites bindendes Protein in der Lage ist, an besagtes MutL Protein oder funktionales Derivat davon zu binden.

18. Ein Set gemäß Anspruch 12, worin besagter Hybridisierungspartner an einem festen Träger, wie beispielsweise eine Nitrocellulose-Membran, immobilisiert ist.

19. Ein Set gemäß Anspruch 12, worin besagtes Fehlpaarungen bindendes Protein ein Fusionsprotein ist, das mit einem Enzym fusioniert ist, und besagtes Reagenz ein chromogenes Substrat für besagtes Enzym umfaßt.

## Revendications

1. Procédé de détection d'une mutation à partir d'une séquence non mutée d'un polynucléotide cible simple-brin dans un échantillon, comprenant :
(a) l'incubation de l'échantillon avec un polynucléotide simple-brin partenaire de l'hybridation comprenant au moins une séquence de bases simple-brin qui est complémentaire de la séquence non mutée du polynucléotide cible, dans des conditions appropriées pour réaliser l'hybridation dudit partenaire d'hybridation avec une quelconque des séquences mutées ou non mutées dudit polynucléotide cible qui peut être présent dans l'échantillon pour former un hybride ;
(b) la mise en présence de l'hybride formé à l'étape (a) avec une protéine de liaison incompatible ; et
(c) la détection de la présence d'une liaison de la protéine de liaison incompatible avec ledit hybride,
la détection de la présence de la protéine de liaison incompatible étant révélatrice de la présence d'une mutation dans la séquence du polynucléotide de l'échantillon.

2. Procédé selon la revendication 1 dans lequel ledit polynucléotide partenaire de l'hybridation est de l'ADN, par exemple de l'ADNc, et/ou ledit polynucléotide cible est de l'ADN.

3. Procédé selon la revendication 1 dans lequel ladite protéine de liaison incompatible est la protéine MutS ou un dérivé fonctionnel de celle-ci.

4. Procédé selon la revendication 1 dans lequel ledit échantillon est un prélèvement ou un fluide biologique qui a été soumis à des conditions suffisantes pour permettre la libération et la dénaturation des acides nucléiques présents dans celui-ci, lesdits acides nucléiques libérés étant éventuellement soumis à une étape de clivage par des endonucléases de restriction préalablement à ladite dénaturation.

5. Procédé selon la revendication 1 dans lequel ledit polynucléotide partenaire de l'hybridation est immobilisé sur un support solide préalablement à ladite incubation de l'étape (a), ledit support solide étant par exemple une membrane de nitrocellulose.

6. Procédé selon la revendication 1 dans lequel ladite étape de détection comprend l'addition d'un premier partenaire de liaison marqué détectable capable de se lier à ladite protéine de liaison incompatible, ledit premier partenaire de liaison étant par exemple un anticorps spécifique de ladite protéine de liaison compatible.

7. Procédé selon la revendication 1 dans lequel ladite étape de détection comprend l'addition d'un premier partenaire de liaison qui n'est pas marqué de manière détectable, ledit premier partenaire de liaison étant capable de se lier à ladite protéine de liaison incompatible et un second partenaire de liaison capable de se lier audit premier partenaire de liaison, et la détection de la présence dudit second partenaire de liaison, ledit second partenaire de liaison étant par exemple un anticorps spécifique dudit premier partenaire de liaison.

8. Procédé selon la revendication 3 dans lequel ladite étape de détection comprend l'addition d'un premier partenaire de liaison et (a) ledit premier partenaire de liaison est une protéine MutL ou un dérivé fonctionnel de celle-ci, pouvant être détecté, ou (b) ledit premier partenaire de liaison est une protéine de fusion entre MutL et une seconde protéine ou un second peptide capable d'être détecté, ladite seconde protéine étant par exemple la béta-galactosidase.

9. Procédé selon la revendication 1 dans lequel ladite protéine de liaison incompatible de l'étape (b) est une protéine de fusion avec une seconde protéine ou un second peptide pouvant d'être détecté, ladite étape de détection comprenant par exemple l'addition d'un premier partenaire de liaison capable de se lier à ladite seconde protéine ou audit second peptide, et la détection de la présence dudit premier partenaire de liaison, ladite seconde protéine étant par exemple la béta-galactosidase.

10. Procédé selon la revendication 9 dans lequel ladite seconde protéine est une enzyme capable de catalyser la génération d'un produit de réaction coloré à partir d'un substrat enzymatique chromogène, et ladite étape de détection comprend l'ajout dudit substrat chromogène et la visualisation dudit produit de réaction coloré.

11. Procédé de détection d'une mutation à partir d'une séquence non mutée d'un polynucléotide cible simple-brin de mammifère dans un échantillon, comprenant :
(a) l'incubation de l'échantillon avec un polynucléotide simple-brin partenaire de l'hybridation comprenant au moins une séquence de bases simple-brin qui est complémentaire de la séquence non mutée du polynucléotide cible de mammifère, dans des conditions appropriées pour réaliser l'hybridation dudit partenaire d'hybridation avec une quelconque des séquences mutées ou non mutées dudit polynucléotide cible de mammifère qui peut être présent dans l'échantillon pour former un hybride ;
(b) mise en présence de l'hybride formé à l'étape (a) avec une protéine de liaison incompatible ; et
(c) détection de la présence d'une liaison de la protéine de liaison incompatible avec ledit hybride,
la détection de la présence de la protéine de liaison incompatible étant révélatrice de la présence d'une mutation dans la séquence du polynucléotide de mammifère de l'échantillon.

12. Kit utile pour la détection d'une mutation à partir d'une séquence non mutée d'une séquence polynucléotide cible dans un échantillon, par un procédé selon l'une quelconque des revendications précédentes, ledit kit étant adapté pour recevoir un ou plusieurs récipients, ledit kit comprenant :
(a) un premier récipient contenant un polynucléotide simple-brin partenaire de l'hybridation comprenant au moins une séquence de bases simple-brin qui est complémentaire de la séquence non mutée du polynucléotide cible ;
(b) un second récipient contenant une protéine de liaison incompatible ; et
(c) un troisième récipient ou une pluralité de récipient contenant un ou des réactif(s) capable(s) de détecter la liaison de ladite protéine de liaison incompatible audit hybride.

13. Kit selon la revendication 12 dans lequel ladite protéine de liaison incompatible est MutS ou un dérivé fonctionnel de celle-ci.

14. Kit selon la revendication 12 dans lequel ledit réactif ou lesdits réactifs comprennent au moins un premier partenaire de liaison capable de se lier à la protéine de liaison incompatible, ledit premier partenaire de liaison étant par exemple un anticorps spécifique de la protéine de liaison incompatible.

15. Kit selon la revendication 12 dans lequel ledit réactif ou lesdits réactifs comprennent un premier partenaire de liaison qui n'est pas marqué de manière détectable, ledit premier partenaire de liaison étant capable de se lier à ladite protéine de liaison incompatible, et un second partenaire de liaison capable de se lier audit premier partenaire de liaison, ledit second partenaire de liaison étant par exemple un anticorps spécifique dudit premier partenaire de liaison.

16. Kit selon la revendication 15 dans lequel ledit premier partenaire de liaison est une protéine MutL ou un dérivé fonctionnel de celle-ci ou est une protéine de fusion entre MutL et une seconde protéine ou peptide capable d'être détectée, ladite seconde protéine étant par exemple la béta-galactosidase.

17. Kit selon la revendication 16 dans lequel ledit second partenaire de liaison est capable de se lier à ladite protéine MutL ou à un dérivé fonctionnel.

18. Kit selon la revendication 12 dans lequel ledit partenaire d'hybridation est immobilisé sur un support solide tel qu'une membrane de nitrocellulose.

19. Kit selon la revendication 12 dans lequel ladite protéine de liaison incompatible est une protéine de fusion avec une enzyme et ledit réactif comprend un substrat chromogène pour ladite enzyme.


### SEQUENCE LISTING

(1) GENERAL INFORMATION:
   (i) APPLICANT:
      Wagner, Jr., Robert
      Radman, Miroslav
   (ii) TITLE OF INVENTION: Method for Detection of Mutations
   (iii) NUMBER OF SEQUENCES: 6
   (iv) CORRESPONDENCE ADDRESS:
      (A) ADDRESSEE: Hamilton, Brook, Smith & Reynolds, P.C.
      (B) STREET: Two Militia Drive
      (C) CITY: Lexington
      (D) STATE: Massachusetts
      (E) COUNTRY: U.S.A.
      (F) ZIP: 02173
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER: US
      (B) FILING DATE:
      (C) CLASSIFICATION:
   (viii) ATTORNEY/AGENT INFORMATION:
      (A) NAME: Granahan, Patricia
      (B) REGISTRATION NUMBER: 32,227
      (C) REFERENCE/DOCKET NUMBER: UBI91-01A
   (ix) TELECOMMUNICATION INFORMATION:
      (A) TELEPHONE: 617-861-6240
      (B) TELEFAX: 617-861-9540
(2) INFORMATION FOR SEQ ID NO:1:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:1:
   CCCCCGCACC TGACTCCTGG GGAGAAGTCT GCCGT 35
(2) INFORMATION FOR SEQ ID NO:2:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: DNA
   (xi) SEOUENCE DESCRIPTION: SEQ ID NO:2:
(2) INFORMATION FOR SEQ ID NO:3:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:3:
   CGTGGACTGA GGACTCCTCT TCAGACGGCA 30
(2) INFORMATION FOR SEQ ID NO:4:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:4:
   CGTGGACTGA GGACGCCTCT TCAGACGGCA 30
(2) INFORMATION FOR SEQ ID NO:5:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:5:
   CGTGGACTGA GGACCCCTCT TCAGACGGCA 30
(2) INFORMATION FOR SEQ ID NO:6:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:6:
   CGTGGACTGA GGACCCCCTC TTCAGACGGC A                31